(19) Europäisches Patentamt
European Patent Office
Office européen des brevets

(11) EP 1 897 876 A2

(12) # EUROPEAN PATENT APPLICATION

(43) Date of publication:
**12.03.2008 Bulletin 2008/11**

(21) Application number: **07122269.9**

(22) Date of filing: **19.06.2003**

(51) Int Cl.:
***C07D 401/04*** (2006.01) ***C07D 215/46*** (2006.01)
***C07D 217/22*** (2006.01) ***A61P 3/00*** (2006.01)
***A61K 31/4706*** (2006.01)

(84) Designated Contracting States:
**AT BE BG CH CY CZ DE DK EE ES FI FR GB GR HU IE IT LI LU MC NL PT RO SE SI SK TR**
Designated Extension States:
**LV**

(30) Priority: **20.06.2002 SE 0201925**
**11.07.2002 SE 0202181**
**26.08.2002 US 406120 P**
**01.10.2002 SE 0202908**
**17.12.2002 US 434010 P**
**10.02.2003 SE 0300357**
**23.04.2003 US 464701 P**

(62) Document number(s) of the earlier application(s) in accordance with Art. 76 EPC:
**03760999.7 / 1 513 828**

(71) Applicant: **Biovitrum AB (publ)**
**112 76 Stockholm (SE)**

(72) Inventors:
- **Johansson, Gary**
  **169 66 Solna (SE)**
- **Jenmalm Jensen, Annika**
  **756 45 Uppsala (SE)**
- **Beierlein, Katarina**
  **756 55 Uppsala (SE)**

Remarks:
•This application was filed on 04-12-2007 as a divisional application to the application mentioned under INID code 62.
•This application was filed on 04-12-2007 as a divisional application to the application mentioned under INID code 62.

(54) **Compounds useful for the treatment of obesity, type II diabetes and CNS disorders**

(57) The present invention relates to compounds of the general formula (I),

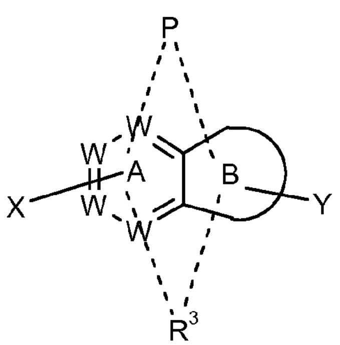

(I)

wherein P is sulfone or sulfonamide; and
A, B, W, X, Y and $R^3$ are as defined in the description; to pharmaceutical compositions comprising these compounds, and to the use of the compounds for the prophylaxis and treatment of medical conditions relating to obesity, type II diabetes, and/or CNS disorders, to achieve reduction of body weight and of body weight gain.

EP 1 897 876 A2

## Description

TECHNICAL FIELD

The present invention relates to substituted sulphone and sulphonamide compounds, to pharmaceutical compositions comprising these compounds, and to the use of the compounds for the prophylaxis and treatment of medical conditions relating to obesity, type 2 diabetes, and/or disorders of the central nervous system (CNS), to achieve reduction of body weight and of body weight gain, as well as for cosmetic use.

BACKGROUND ART

Obesity is a condition characterized by an increase in body fat content resulting in excess body weight above accepted norms. Obesity is the most important nutritional disorder in the western world and represents a major health problem in all industrialized countries. This disorder leads to increased mortality due to increased incidences of diseases such as cardiovascular disease, digestive disease, respiratory disease, cancer and type 2 diabetes. Searching for compounds, which reduce body weight has been going on for many decades. One line of research has been activation of serotoninergic systems, either by direct activation of serotonin receptor subtypes or by inhibiting serotonin reuptake. The exact receptor subtype profile required is however not known.

Serotonin (5-hydroxytryptamine or 5-HT), a key transmitter of the peripheral and central nervous system, modulates a wide range of physiological and pathological functions, including anxiety, sleep regulation, aggression, feeding and depression. Multiple serotonin receptor subtypes have been identified and cloned. One of these, the 5-$HT_6$ receptor, was cloned by several groups in 1993 (Ruat, M. et al. (1993) Biochem. Biophys. Res. Commun.193: 268-276; Sebben, M. et al. (1994) NeuroReport 5: 2553-2557). This receptor is positively coupled to adenylyl cyclase and displays affinity for antidepressants such as clozapine. Recently, the effect of 5-$HT_6$ antagonist and 5-$HT_6$ antisense oligonucleotides to reduce food intake in rats has been reported (Bentley, J.C. et al. (1999) Br J Pharmac. Suppl. 126, P66; Bentley, J.C. et al. (1997) J. Psychopharmacol. Suppl. A64, 255; Woolley M.L. et al. (2001) Neuropharmacology).

Compounds with enhanced affinity and selectivity for the 5-$HT_6$ receptor have been identified, e.g. in WO 00/34242 and by Isaac, M. et al. (2000) 6-Bicyclopiperazinyl-1-arylsulfonylindoles and 6-Bicyclopiperidinyl-1-arylsulfonylindoles derivatives as novel, potent and selective 5-HT6 receptor antagonists. Bioorganic & Medicinal Chemistry Letters 10: 1719-1721 (2000).

INFORMATION DISCLOSURE

J. Med. Chem. 1970, 13(4), 592-598 describes N-(4-{[2-(diethylamino)ethyl]amino}-1-naphthyl)amides; N-{5,6,7,8-Tetrahydro-4-[(3-piperidinopropyl)amino]-1-naphthyl} amides and related amides and urea derivatives as schistosomicides.

WO 99/42465 discloses sulphonamides derivatives that bind to the 5-$HT_6$ receptor and that can be used for the treatment of CNS disorders such as anxiety, depression, epilepsy, obsessive compulsive disorders, cognitive disorders, ADHD, anorexia and bulimia schizophrenia, drug abuse.

WO 01/32646 A1 discloses compounds that bind to the 5-$HT_6$ receptor and that are used for the treatment of CNS disorders and which inter alia may be used for the treatment of eating disorders.

WO 99/37623 A2 discloses compounds that bind to the 5-$HT_6$ receptor and that are used for the treatment of CNS disorders and which inter alia may be used for the treatment of eating disorders.

WO 99/42465 A3 discloses compounds that bind to the 5-$HT_6$ receptor and that are used for the treatment of CNS disorders and which inter alia may be used for the treatment of eating disorders.

EP 0 815 861 A1 discloses compounds that bind to the 5-$HT_6$ receptor and that are used for the treatment of CNS disorders.

WO 99/02502 A2 discloses compounds that bind to the 5-$HT_6$ receptor and that are used for the treatment of CNS disorders and which inter alia may be used for the treatment of eating disorders.

WO 98/27081 A1 discloses compounds that bind to the 5-$HT_6$ receptor and that are used for the treatment of CNS disorders and which inter alia may be used for the treatment of eating disorders.

EP 0701819 discloses compounds that bind to the 5-$HT_{1D}$ receptor and that are used for the treatment of CNS disorders and obesity.

US 6,191,141 and WO 01/12629 disclose compounds that bind to the 5-$HT_6$ receptor and that are used for the treatment of CNS disorders.

DISCLOSURE OF THE INVENTION

It has surprisingly been found that the compounds of formula (I) show affinity for the 5-$HT_6$ receptor as antag-

onists at low nanomolar range. Compounds according to the invention and their pharmaceutically acceptable salts have 5-$HT_6$ receptor antagonist, agonist and partial agonist activity and are believed to be of potential use in the treatment or prophylaxis of obesity and type 2 diabetes, to achieve reduction of body weight and of body weight gain, as well as in the treatment or prophylaxis of disorders of the central nervous system such as anxiety, depression, panic attacks, memory disorders, cognitive disorders, sleep disorders, migraine, anorexia, bulimia, binge disorders, obsessive compulsive disorders, psychoses, Alzheimer's disease, Parkinson's disease, Huntington's chorea and/or schizophrenia, Attention Deficit Hyperactive Disorders (ADHD), drug abuse. The reduction of body weight and of body weight gain (e.g. treating body-weight disorders) is achieved *inter alia* by reduction of food intake. As used herein, the term "body weight disorders" refers to the disorders caused by an imbalance between energy intake and energy expenditure, resulting in abnormal body (e.g., excessive) weight. Such body weight disorders include obesity.

*Definitions*

Unless otherwise stated or indicated, the term "$C_{1-6}$ alkyl" (or "$C_{2-6}$ alkenyl") denotes a straight or branched hydrocarbon chain group having from 1 to 6 carbon atoms (or 2 to 6 carbon atoms). Examples of said lower alkyl include methyl, ethyl, n-propyl, iso-propyl, n-butyl, iso-butyl, sec-butyl, t-butyl and straight- and branched-chain pentyl and hexyl. Alkenyl groups have one or more double carbon-carbon bonds in the chain.

Unless otherwise stated or indicated, the term "$C_{1-6}$ alkoxy" denotes a straight or branched alkoxy group having from 1 to 6 carbon atoms. Examples of said lower alkoxy include methoxy, ethoxy, n-propoxy, iso-propoxy, n-butoxy, iso-butoxy, sec-butoxy, t-butoxy and straight- and branched-chain pentoxy and hexoxy.

Unless otherwise stated or indicated, the term "$C_{1-6}$ alkoxyalkyl" denotes a straight or branched alkoxyalkyl group having from 1 to 6 carbon atoms. Examples of said lower alkoxyalkyl include methoxymethyl, ethoxymethyl, iso-propoxymethyl, n-butoxymethyl, t-butoxyethyl and straight- and branched-chain pentoxymethyl.

The expression "$C_{2-6}$ alkenyl" as used herein refers to straight-chained and branched alkenyl groups containing from 2 to 6 carbon atoms. Typical examples include vinyl, allyl, 2,3-dimethylallyl, 1-butenyl groups, 1-pentenyl, and 1-hexenyl groups.

The expression "$C_{2-6}$ alkynyl" as used herein refers to straight-chained and branched alkynyl groups containing from 2 to 6 carbon atoms. Typical examples include ethynyl, 1-propynyl, 1-butynyl, 1-pentynyl, and 1-hexynyl groups.

Unless otherwise stated or indicated, the term "halogen" shall mean fluorine, chlorine, bromine or iodine.

The term "alkylhalide" refers to an alkyl group substituted with one or more halogen groups (e.g., F, Cl, Br, I).

The term "$C_{3-7}$ cycloalkyl" denotes a cyclic alkyl group having a ring size from $C_3$ to $C_7$, which can be saturated or partially unsaturated. Examples of said cycloalkyl include cyclopropyl, cyclobutyl, cyclopentyl, cyclopentenyl, cyclopentadienyl, cyclohexyl, methylcyclohexyl, cyclohexenyl, cyclohexadienyl, and cycloheptyl.

The term "$C_{5-10}$ cycloalkenyl" denotes a cyclic alkenyl group having a ring size from $C_5$ to $C_{10}$. Examples of said cycloalkenyl include 1-cyclopentyl, 2-cyclopentenyl, 1-cyclohexenyl, 1-cyclohepentyl, 1-cyclooctenyl, 1-cyclononenyl, and 1-cyclodecenyl groups.

The term "heterocyclic" refers to a hydrocarbon ring system containing 4 to 8 ring members that have at least one heteroatom (e.g., S, N, or O) as part of the ring. It includes saturated, unsaturated, aromatic, and nonaromatic heterocycles. Suitable heterocyclic groups include thienyl, furyl, pyridyl, pyrrolidinyl, imidazolyl, pyrazolyl, piperidyl, azepinyl, morpholinyl, pyranyl, dioxanyl, pyridazinyl, pyrimidinyl, and piperazinyl groups

Unless otherwise stated or indicated, the term "aryl" refers to a hydrocarbon ring system having at least one aromatic ring. Examples of aryl groups include phenyl, cinnamyl, pentalenyl, indenyl, 1-naphthyl, 2-naphthyl, anthryl and phenanthryl.

The term "heteroaryl" refers to a hydrocarbon ring system having at least one aromatic ring which contains at least one heteroatom such as O, N, or S. Examples of heteroaryl groups include furyl, pyrrolyl, thienyl, oxazolyl, imidazolyl, thiazolyl, pyridinyl, pyrimidinyl, quinazolinyl, and indolyl groups.

*Compounds of Formula (I)*

One object of the present invention is a compound having the general formula (I):

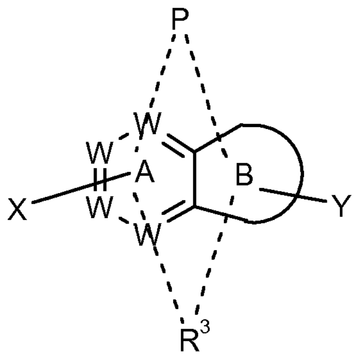

(I)

or a pharmaceutically acceptable salt thereof, wherein:
ring B is

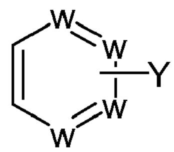

;

each W is independently -N-, -(CH)-, or -C- provided that not more than three groups W are -N- in both rings A and B together; and that rings A and B are not both phenyl;
P is any one of formula (a), (b) or (c)

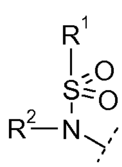

,

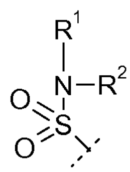

, or

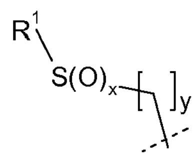

;

(a) (b) (c)

wherein x = 0, 1, or 2 and y = 0, 1, or 2;
and P and $R^3$ can be attached to any carbon atom that allows the substitution in one of either the A- or B-ring, or when ring A contains at least one nitrogen atom and P is (c), then P can also be attached to any nitrogen in ring B that allows the substitution;
the dashed bonds denote that P and $R^3$, respectively, may be attached to either the A or B ring; but each P or $R^3$ may not be simultaneously bound to both rings A and B;
$R^1$ is

(a) $C_{1-6}$ alkyl,
(b) $C_{1-6}$ alkoxyalkyl,
(c) straight-chained or branched $C_{1-6}$ hydroxyalkyl,
(d) straight-chained or branched $C_{1-6}$ alkylhalides,
(e) aryl carbonylmethyl,
(f) $C_{3-7}$ cycloalkyl, which is optionally partially unsaturated,
(g) $C_{3-7}$ cycloalkyl-$C_{1-6}$ alkyl, wherein the cyclic ring is optionally partially unsaturated, or
(h) a group Ar;

wherein Ar is

(a) phenyl,
(b) 1-naphthyl,
(c) 2-naphthyl,
(d) aryl-$C_{1-6}$ alkyl,
(e) cinnamyl,
(f) a 5 to 7-membered, optionally aromatic, partially saturated or completely saturated, mono- or bi-cyclic heterocyclic ring, each containing 1 to 4 heteroatoms, selected from oxygen, sulfur, and nitrogen,
(g) a bicyclic ring system comprising at least one heterocyclic ring according to (f) and a group Ar,
wherein the group Ar is substituted in one or more positions with

(a) H, X or Y, or
(b) a 5 to 7-membered, optionally aromatic, partially saturated or completely saturated, heterocyclic ring each containing 1 to 4 heteroatoms selected from oxygen, nitrogen or sulfur;

$R^2$ is

(a) H,
(b) $C_{1-6}$ alkyl,
(c) $C_{2-6}$ alkoxyalkyl,
(d) straight or branched $C_{1-6}$ hydroxyalkyl, or
(e) straight or branched $C_{1-6}$ alkylhalides;
(f) a group Ar,

or $R^1$ and $R^2$ are linked to form a group $-CH_2CH_2OCH_2CH_2-$ or

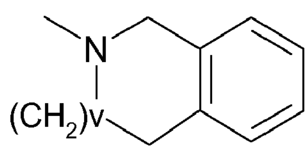

wherein v is 0-2,
X and Y are independently

(a) H,
(b) halogen,
(c) $C_{1-6}$ alkyl,
(d) $CF_3$,
(e) hydroxy,
(f) $C_{1-6}$ alkoxy,
(g) $C_{2-6}$ alkenyl,
(h) phenyl,
(i) phenoxy,
(j) benzyloxy,
(k) benzoyl,
(l) $-OCF_3$,
(m)-CN,
(n) straight or branched $C_{1-6}$ hydroxyalkyl,
(o) straight or branched $C_{1-6}$ alkylhalides,
(p) $-NH_2$,
(q) $-NHR^4$,
(r) $-NR^4R^5$,
(s) $-NO_2$,
(t) $-CONR^4R^5$,
(u) $-NHSO_2R^4$,

(v) $-NR^4COR^5$,
(x) $-SO_2NR^4R^5$,
(z) $-C(=O)R^4$,
(aa) $-CO_2R^4$, or
(ab) $-S(O)_nR^4$, wherein n is 0, 1, 2 or 3,
(ac) $-S-(C_{1-6})$ alkyl, or
(ad) $-SCF_3$; and

$R^4$ and $R^5$ are independently

(a) H,
(b) $C_{1-6}$ alkyl,
(c) $C_{3-7}$ cycloalkyl, or
(d) Ar, as defined above for $R^1$;

alternatively, $R^4$ and $R^5$ are linked to form a group $-CH_2OCH_2-$, $-CH_2CH_2OCH_2CH_2-$ or $(CH_2)_{3-5}$;
$R^3$ is a group selected from any one of

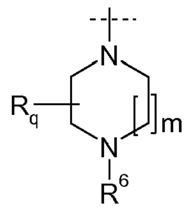
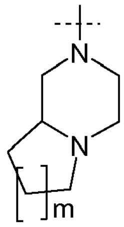
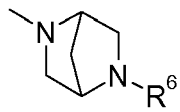
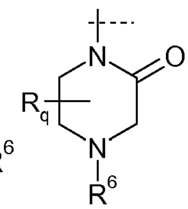
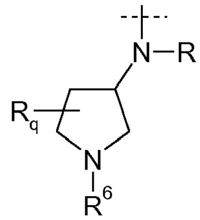

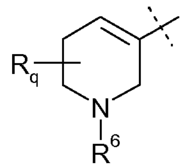
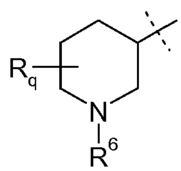
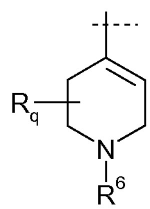
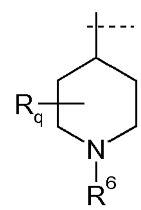
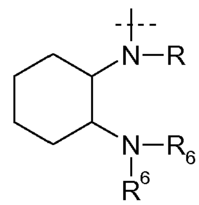

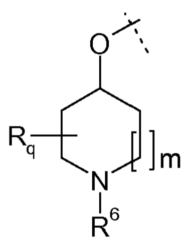
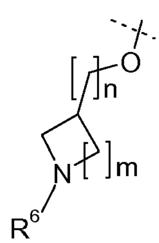
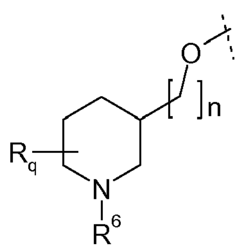
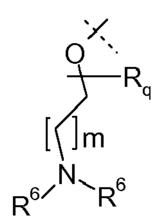

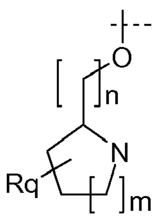
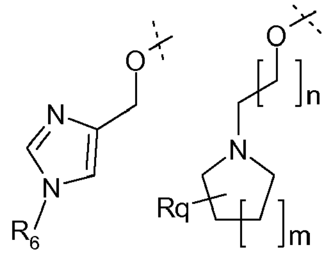
and
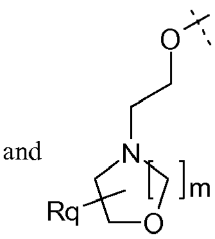

wherein $R^3$ is optionally substituted on each carbon atom that allows the substitution with Rq groups, wherein Rq is independently H, or $(C_{1-6})$ alkyl, and wherein two Rq groups can be present on the same carbon atom simultaneously, wherein

q = 1, 2, 3, 4, 5 or 6,

m = 1 or 2, and

n = 0, 1 or 2;

$R^6$ is independently

(a) H,

(b) linear or branched $C_{1-6}$ alkyl,

(c) benzyl,

(d) $-CH_2-CH_2-OH$, or

(e) $-CH_2-CH_2-O-C_{1-6}$ alkyl;

P and $R^3$ can be attached to the same ring or to different rings of rings A and B; provided that when P is

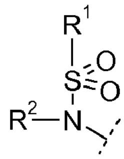
(a) or
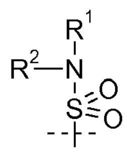

(b), and P and $R^3$ both are attached to ring A in the meta- or para-position relative to one another then $R^3$ is selected from any one of

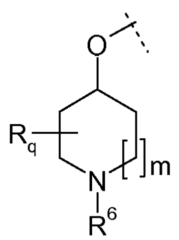
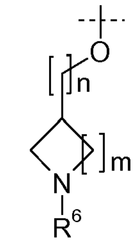
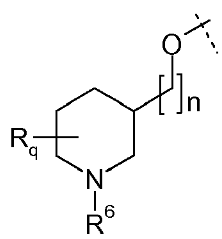
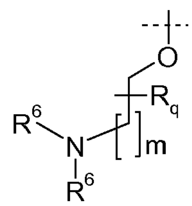

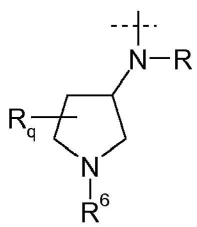
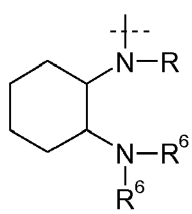
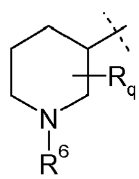
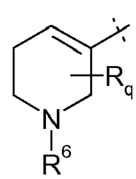

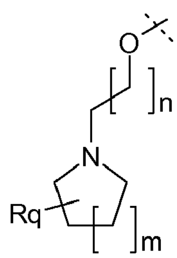
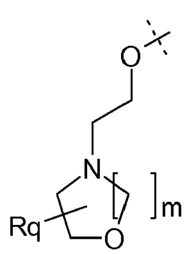
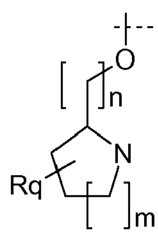
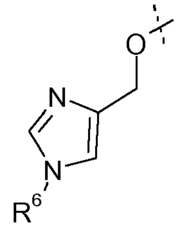
;

when P is

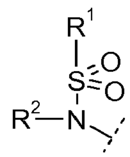

(a), then P and $R^3$ are simultaneously attached to the same ring A or B;
when P is

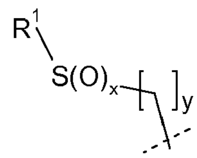

,

wherein y = 0, then P and $R^3$ are attached to the different rings of rings A and B; or
when $R^1$ = Ar is partially saturated bi-cyclic heterocyclic ring containing a N atom, the N atom in Ar cannot be attached to the S atom in.

It is preferred that:

$R^1$ is

(a) $C_{1-6}$ alkyl, or
(e) a group Ar;

Ar is

(a) phenyl,
(b) 1-naphthyl,
(c) 2-naphthyl, or
(f) a 5 to 7-membered, optionally aromatic, partially saturated or completely saturated, heterocyclic ring containing 1 to 4 heteroatoms, selected from oxygen, nitrogen and sulfur,

wherein the group Ar is substituted in one or more positions with

(a) H,
(b) halogen,
(c) $C_{1-6}$ alkyl,
(d) $-CF_3$,
(f) $C_{1-6}$ alkoxy,
(g) $C_{2-6}$ alkenyl (preferably $C_{2-4}$ alkenyl),
(l) $-OCF_3$,
(m) straight or branched $C_{1-6}$ hydroxyalkyl,
(n) phenyloxy,
(o) benzyloxy,
(v) $-NR^4COR^5$,
(x) $-SO_2NR^4R^5$,
(z) $-C(=O)R^4$,
(ab) $-S(O)_nR^4$, wherein n is 0, 1, 2 or 3;

(ac) -S-($C_{1-6}$) alkyl, or
(ad) $-SCF_3$;

$R^2$ is

(a) H, or
(b) $C_{1-6}$ alkyl;

or $R^1$ and $R^2$ are linked to form a group $-CH_2CH_2OCH_2CH_2-$;
X and Y are H;
$R^4$ and $R^5$ are each independently H or $C_{1-3}$ alkyl; and
$R^3$ is selected from any one of

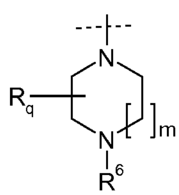
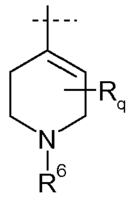
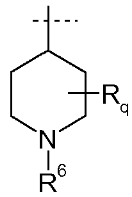

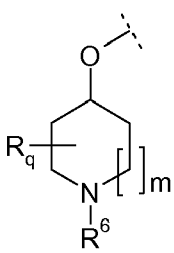
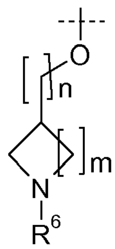
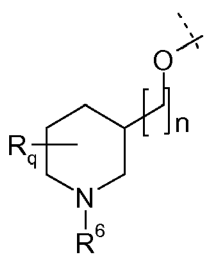
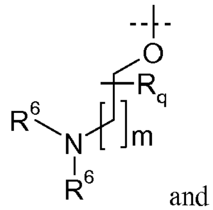
and
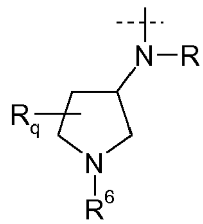

wherein $R^3$ can be substituted on each carbon atom that allows the substitution with Rq groups, wherein Rq is independently H, or $C_{1-6}$ alkyl, and wherein two Rq groups can be present on the same carbon atom simultaneously, wherein
q = 1 or 2,
m = 1 or 2,
n = 0, and
$R^6$ is independently

(a) H,
(b) $C_{1-6}$ alkyl (preferably $C_{1-3}$ alkyl), in particular methyl,
(d) $-CH_2-CH_2-OH$, or
(e) $-CH_2-CH_2-OCH_3$.

It is especially preferred that $R^3$ is selected from any one of

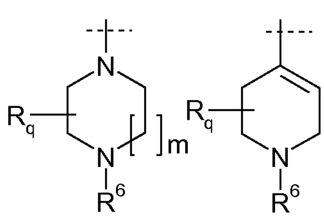
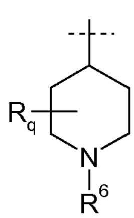
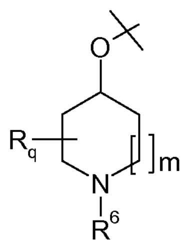
and
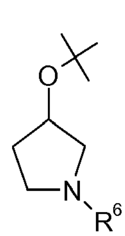

wherein $R^3$ can be substituted on each carbon atom that allows the substitution with Rq groups, wherein Rq is inde-

pendently H, or $C_{1-2}$ alkyl, and wherein two Rq groups can be present on the same carbon atom simultaneously, wherein q = 1 or 2,
m = 1 or 2; and
$R^6$ is independently

(a) H,
(b) $C_{1-3}$ alkyl,
(d) $-CH_2-CH_2-OH$, or
(e) $-CH_2-CH_2-OCH_3$.

It is also preferred that $R^3$ is selected from any one of

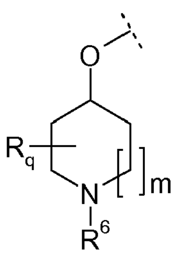
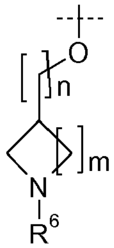
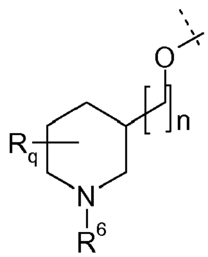
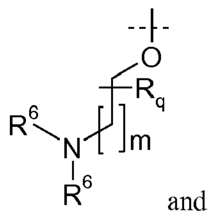
and
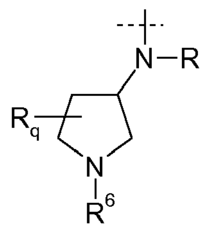

wherein $R^3$ can be substituted on each carbon atom that allows the substitution with Rq groups, wherein Rq is independently H, or $C_{1-6}$ alkyl, and wherein two Rq groups can be present on the same carbon atom simultaneously, wherein q = 1 or 2,
m = 1 or 2,
n = 0, and
$R^6$ is independently

(a) H,
(b) $C_{1-3}$ alkyl,
(d) $-CH_2-CH_2-OH$, or
(e) $-CH_2-CH_2-OCH_3$.

It is also preferred that $R^3$ is selected from any one of

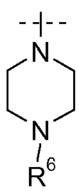 ; 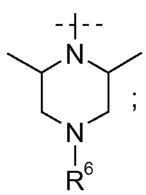 ; 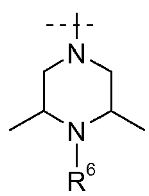 ; 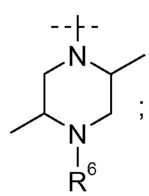 ; 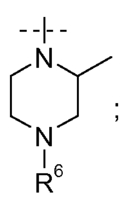 ;

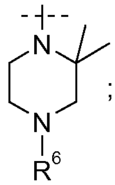 ; 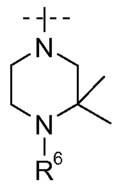 ; 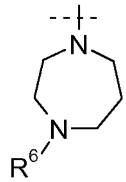 ; 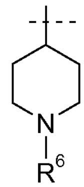 ; 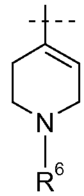 ;

$R^6$ is independently

(a) H,
(b) $C_{1-3}$ alkyl,
(d) $-CH_2-CH_2-OH$, or
(e) $-CH_2-CH_2-OCH_3$.

It is preferred that $R^6$ is H or methyl.

It is also preferred that $R^3$ is piperazine; homopiperazine; 2,6-dimethylpiperazine; 3,5-dimethylpiperazine; 2,5-dimethylpiperazine; 2-methylpiperazine; 3-methylpiperazine; 2,2-dimethylpiperazine; 3,3-dimethylpiperazine; piperidine; 1,2,3,6-tetrahydro-pyrazine; or 4-pyrrolidin-3-yloxy.

It is preferred that the groups Y and X are attached to any unsubstituted carbon atom.

It is preferred that P is

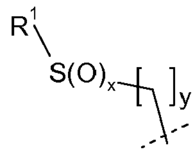

(c)

wherein $R^1$, x, and y are as defined in claim 1.

It is also preferred that P is

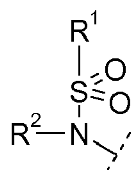

(a) or

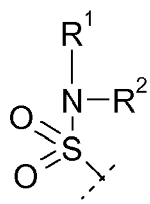

(b)

wherein $R^1$ and $R^2$ are as defined in claim 1.

It is preferred that $R^2$ is H.

Another object of the present invention is a compound of the general formula (II)

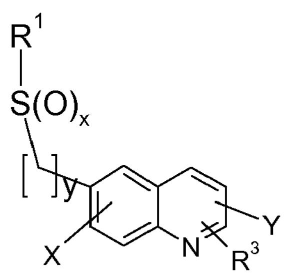

(II)

wherein $R^1$, x, y, X, and Y are as defined in claim 1, and $R^3$ is as defined in claim 2.

It is preferred that y = 0 and x = 2.

Another object of the present invention is a compound of the general formula (III)

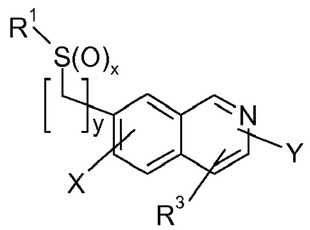

(III)

wherein $R^1$, x, y, X, and Y are as defined in claim 1, and $R^3$ is as defined in claim 2.

It is preferred that y = 0 and x = 2

Preferred compounds of the formula (II) are
6-Benzenesulfonyl-4-piperazin-1-yl-quinoline hydrochloride;
6-[(2-Fluorophenyl)sulfonyl]-4-piperazin-1-ylquinoline hydrochloride;
6-(1-Naphthylsulfonyl)-4-piperazin-1-ylquinoline hydrochloride;
6-[(3,4-Dichlorophenyl)sulfonyl]-4-piperazin-1-ylquinoline hydrochloride;
6-[(3,5-Dimethylphenyl)sulfonyl]-4-piperazin-1-ylquinoline hydrochloride;
6-[(2-Chloro-6-methylphenyl)sulfonyl]-4-piperazin-1-ylquinoline hydrochloride;
6-[(4-Chlorophenyl)sulfonyl]-4-piperazin-1-ylquinoline hydrochloride;
6-[(2-Methyl,4-tert-butyl-phenyl)sulfonyl]-4-piperazin-1-ylquinoline hydrochloride;
6-[(3,4-Dimethylphenyl)sulfonyl]-4-piperazin-1-ylquinoline hydrochloride;
6-[(2,3-Dichlorophenyl)sulfonyl]-4-piperazin-1-ylquinoline hydrochloride;
6-[(4-tert-Butylphenyl)sulfonyl]-4-piperazin-1-ylquinoline hydrochloride;
6-[(4-Isopropylphenyl)sulfonyl]-4-piperazin-1-ylquinoline hydrochloride;
(4-Piperazin-1-yl-6-{[4-(trifluoromethyl)phenyl]sulfonyl}quinoline hydrochloride;
6-[(4-tert-Butylphenyl)sulfonyl]-4-(1,4-diazepan-1-yl)quinoline hydrochloride; and
4-(1,4-Diazepan-1-yl)-6-[(4-isopropylphenyl)sulfonyl]quinoline hydrochloride.

Preferred compounds of the formula (III) are
7-(2-Chloro-6-methyl-benzenesulfonyl)-1-piperazin-1-yl-isoquinoline hydrochloride;
7-(2-*t*-Butyl-benzenesulfonyl)-1-piperazin-1-yl-isoquinoline hydrochloride;
7-(3,4-Dichloro-benzenesulfonyl)-1-piperazin-1-yl-isoquinoline hydrochloride;
7-(2,4-Dimethyl-benzenesulfonyl)-1-piperazin-1-yl-isoquinoline hydrochloride;
7-(2,5-Dimethyl-benzenesulfonyl)-1-piperazin-1-yl-isoquinoline hydrochloride;
7-(*p*-Chloro-benzenesulfonyl)-1-piperazin-1-yl-isoquinoline hydrochloride;
7-Benzenesulfonyl-1-[1,4]diazepan-1-yl-isoquinoline,hydrochloride;
7-(4-tert-Butyl-benzenesulfonyl)-1-[1,4]diazepan-1-yl]-isoquinoline, hydrochloride;
7-(2-Chloro-6-methyl-benzenesulfonyl)-1-[1,4]diazepan-1-yl]-isoquinoline hydrochloride;
7-(3,5-Dimethyl-benzenesulfonyl)-1-[1,4]diazepan-1-yl]-isoquinoline hydrochloride;
7-(3,4-Dichloro-benzenesulfonyl)-1-[1,4]diazepan-1-yl]-isoquinoline hydrochloride;
7-(4-Chloro-benzenesulfonyl)-1-[1,4]diazepan-1-yl]-isoquinoline hydrochloride;
7-(3,4-Dimethyl-benzenesulfonyl)-1-[1,4]diazepan-1-yl]-isoquinoline hydrochloride;
7-(2-tert-Butyl-benzenesulfonyl)-1-1,4]diazepan-1-yl]-isoquinoline hydrochloride;
7-Benzenesulfonyl-1-piperazin-yl-isoquinoline hydrochloride; and
7-(4-tert-Butyl-benzenesulfonyl-1-piperazin-yl-isoquinoline hydrochloride

All diastereomeric forms possible (pure enantiomers, tautomers, racemic mixtures and unequal mixtures of two enantiomers) are within the scope of the invention. Such compounds can also occur as cis- or trans-, *E*- or *Z*- double bond isomer forms. All isomeric forms are contemplated.

The compounds of the formulae (I) to (III) may be used as such or, where appropriate, as pharmacologically acceptable salts (acid or base addition salts) thereof.

The pharmacologically acceptable addition salts as mentioned above are meant to comprise the therapeutically active non-toxic acid and base addition salt forms that the compounds are able to form. Compounds that have basic properties can be converted to their pharmaceutically acceptable acid addition salts by treating the base form with an appropriate acid. Exemplary acids include inorganic acids, such as hydrogen chloride, hydrogen bromide, hydrogen iodide, sulphuric acid, phosphoric acid; and organic acids such as acetic acid, propanoic acid, hydroxyacetic acid, lactic acid, pyruvic acid, glycolic acid, maleic acid, malonic acid, oxalic acid, benzenesulphonic acid, toluenesulphonic acid, methanesulphonic acid, trifluoroacetic acid, fumaric acid, succinic acid, malic acid, tartaric acid, citric acid, salicylic acid, p-aminosalicylic acid, pamoic acid, benzoic acid, ascorbic acid and the like. Exemplary base addition salt forms are the sodium, potassium, calcium salts, and salts with pharmaceutically acceptable amines such as, for example, ammonia, alkylamines, benzathine, and amino acids, such as, e.g. arginine and lysine. The term addition salt as used herein also comprises solvates which the compounds and salts thereof are able to form, such as, for example, hydrates, alcoholates and the like.

For clinical use, the compounds of the invention are formulated into pharmaceutical formulations for oral, rectal, parenteral or other mode of administration. Pharmaceutical formulations are usually prepared by mixing the active substance, or a pharmaceutically acceptable salt thereof, with conventional pharmaceutical excipients. The formulations can be further prepared by known methods such as granulation, compression, microencapsulation, spray coating, etc. The formulations may be prepared by conventional methods in the dosage form of tablets, capsules, granules, powders, syrups, suspensions, suppositories or injections. Liquid formulations may be prepared by dissolving or suspending the active substance in water or other suitable vehicles. Tablets and granules may be coated in a conventional manner.

Another object of the present invention is a compound above for use in therapy.

Another object of the present invention is a compound above for use in the treatment or prophylaxis of a 5-$HT_6$ receptor related disorder, such as obesity, type II diabetes, and/or disorders of the central nervous system, to achieve reduction of body weight and of body weight gain.

Another object of the present invention is a compound above for use in the treatment or prophylaxis of disorders of the central nervous system.

Another object of the present invention is a compound above for use in the treatment or prophylaxis of type II diabetes.

Another object of the present invention is a compound above for use in the treatment or prophylaxis of obesity, to achieve reduction of body weight and of body weight gain.

Another object of the present invention is a pharmaceutical formulation comprising a compound above as an active ingredient, in combination with a pharmaceutically acceptable diluent or carrier.

Another object of the present invention is a pharmaceutical formulation comprising a compound above as an active ingredient, for use in the treatment or prophylaxis of a 5-$HT_6$ receptor related disorder, such as obesity, type II diabetes, and/or disorders of the central nervous system, to achieve reduction of body weight and of body weight gain.

Another object of the present invention is a compound above as an active ingredient, for use in the treatment or prophylaxis of disorders of the central nervous system.

Another object of the present invention is a pharmaceutical formulation comprising a compound above as an active ingredient, for use in the treatment or prophylaxis of type II diabetes.

Another object of the present invention is a pharmaceutical formulation comprising a compound above as an active ingredient, for use in the treatment or prophylaxis of obesity, to achieve reduction of body weight and of body weight gain.

Another object of the present invention is a method for the treatment or prophylaxis of a 5-$HT_6$ receptor related disorder, such as obesity, type II diabetes, and/or disorders of the central nervous system, to achieve reduction of body weight and of body weight gain, which comprises administering to a subject (e.g., a mammal, a human, a horse, a dog, or a cat) in need of such treatment an effective amount of one or more compounds of any of the formulae described above, their salt forms or compositions that include the compounds or their salt forms.

Another object of the present invention is a method for the treatment or prophylaxis of disorders of the central nervous system, which comprises administering to a subject in need of such treatment an effective amount of one or more compounds of any of the formulae described above, their salt forms or compositions that include the compounds or their salt forms.

Another object of the present invention is a method for the treatment or prophylaxis of type II diabetes, which comprises administering to a subject in need of such treatment an effective amount of one or more compounds of any of the formulae described above, their salt forms or compositions that include the compounds or their salt forms.

Another object of the present invention is a method for the treatment or prophylaxis of obesity, which comprises administering to a subject in need of such treatment an effective amound of one or more compounds of any of the formulae described above, their salt forms or compositions that include the compounds or their salt forms.

Another object of the present invention is a method for modulating 5-$HT_6$ receptor activity, comprising administering to a subject in need thereof an effective amount of one or more compounds of any of the formulae described above, their salt forms or compositions that include the compounds or their salt forms.

Another object of the present invention is the use of one or more compounds of any of the formulae described above, their salt forms or compositions that include the compounds or their salt forms, for the manufacture of a medicament for use in the treatment or prophylaxis of a 5-$HT_6$ receptor related disorder, such as obesity, type II diabetes, and/or disorders of the central nervous system, to achieve reduction of body weight and of body weight gain.

Another object of the present invention is the use of one or more compounds of any of the formulae described

above, their salt forms or compositions that include the compounds or their salt forms for the manufacture of a medicament for use in the treatment or prophylaxis of disorders of the central nervous system.

Another object of the present invention is the use of one or more compounds of any of the formulae described above, their salt forms or compositions that include the compounds or their salt forms for the manufacture of a medicament for use in the treatment or prophylaxis of type II diabetes.

Another object of the present invention is the use of one or more compounds of any of the formulae described above, their salt forms or compositions that include the compounds or their salt forms for the manufacture of a medicament for use in the treatment or prophylaxis of obesity, to achieve reduction of body weight and of body weight gain.

The methods delineated herein can also include the step of identifying that the subject is in need of treatment of obesity, type II diabetes, or disorders of the central nervous system, or in need of reducing body weight and of body weight gain.

The invention further relates to cosmetic use of one or more compounds of any of the formulae described herein, for causing loss of weight, as well as cosmetic compositions containing said compounds.

Still further, the invention relates to a non-therapeutic metod for impriving the bodily appearance of a mammal, including a human, in which the method comprises orally administering to said mammal one or more compounds of any of the formulae described herein.

"An effective amount" refers to an amount of a compound that confers a therapeutic effect on the treated subject. The therapeutic effect may be objective (i.e., measurable by some test or marker) or subjective (i.e., subject gives an indication of or feels an effect).

For clinical use, the compounds of the invention are formulated into pharmaceutical formulations for oral, rectal, parenteral or other mode of administration. Usually the amount of active compounds is between 0.1-95% by weight of the preparation, preferably between 0.2-20% by weight in preparations for parenteral use and preferably between 1 and 50% by weight in preparations for oral administration.

The typical daily dose of the active substance varies within a wide range and will depend on various factors such as, for example, the individual requirement of each patient and the route of administration. In general, oral and parenteral dosages will be in the range of 5 to 1000 mg per day of active substance, preferably 50 to 150 mg per day.

***Processes for preparation***

In a further aspect the invention relates to methods of making compounds of any of the formulae herein comprising reacting any one or more of the compounds of the formulae delineated herein, including any processes delineated herein. The compounds of the formulae above may be prepared by, or in analogy with, conventional methods, and especially according to or in analogy with the following methods.

The chemicals used in the above-described synthetic route may include, for example, solvents, reagents, catalysts, protecting group and deprotecting group reagents. The methods described above may also additionally include steps, either before or after the steps described specifically herein, to add or remove suitable protecting groups in order to ultimately allow synthesis of the compounds of any of the formulae described above, their salt forms, or compositions that include the compounds or their salt forms. In addition, various synthetic steps may be performed in an alternate sequence or order to give the desired compounds. Synthetic chemistry transformations and protecting group methodologies (protection and deprotection) useful in synthesizing applicable compounds are known in the art and include, for example, those described in R. Larock, Comprehensive Organic Transformations, VCH Publishers (1989); T.W. Greene and P.G.M. Wuts, Protective Groups in Organic Synthesis, 2nd Ed., John Wiley and Sons (1991); L. Fieser and M. Fieser, Fieser and Fieser's Reagents for Organic Synthesis, John Wiley and Sons (1994); and L. Paquette, ed., Encyclopedia of Reagents for Organic Synthesis, John Wiley and Sons (1995) and subsequent editions thereof.

The specific examples below are to be construed as merely illustrative, and not limitative of the remainder of the disclosure in any way whatsoever. Without further elaboration, it is believed that one skilled in the art can, based on the description herein, utilize the present invention to its fullest extent. All publications cited herein are hereby incorporated by reference in their entirety.

**Methods**

$^{1}H$ nuclear magnetic resonance (NMR) and $^{13}C$ NMR were recorded on a Bruker Advance DPX 400 spectrometer at 400.1 and 100.6 MHz, respectively. All spectra were recorded using residual solvent or tetramethylsilane (TMS) as internal standard. IR spectra were recorded on a Perkin-Elmer Spectrum 1000 FT-IR spectrophotometer. Ionspray mass spectrometry (MS) spectra were obtained on a Perkin-Elmer API 150EX mass spectrometer. Accurate mass measurements were performed on a Micromass LCT dual probe. Preparative HPLC/MS was performed on a Waters/ Micromass Platform ZQ system equipped with System A: ACE 5 C8 column (19x50mm), eluents: MilliQ water, MeCN and MilliQ/MeCN/0.1%TFA and system B: Xterra MS C18, 5μm column (19x50mm), eluents: MilliQ water, MeCN and

$NH_4HCO_3$ (100mM). Analytical HPLC were performed on Agilent 1100, column: ACE 3 C8 (system A) or column: YMC-Pack (system B), eluents: MilliQ/0.1%TFA and MeCN. Elemental analyses were performed on a Vario El instrument. Preparative flash chromatography was performed on Merck silica gel 60 (230-400 mesh).

**Table 1**

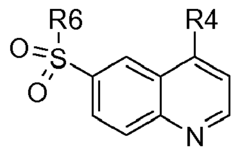

| EXAMPLE | | $R^6$ | $R^4$ |
|---|---|---|---|
| 1 | 6-Benzenesulfonyl-4-piperazin-1-yl-quinoline hydrochloride | 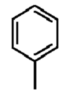 | 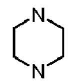 |
| 2 | 6-[(2-Fluorophenyl)sulfonyl]-4-piperazin-1-ylquinoline hydrochloride | 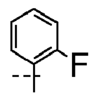 | 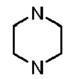 |
| 3 | 6-(1-Naphthylsulfonyl)-4-piperazin-1-ylquinoline hydrochloride | 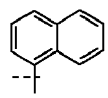 | 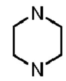 |
| 4 | 6-[(3,4-Dichlorophenyl)sulfonyl]-4-piperazin-1-ylquinoline hydrochloride | 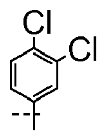 | 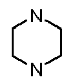 |
| 5 | 6-[(3,5-Dimethylphenyl)sulfonyl]-4-piperazin-1-ylquinoline hydrochloride | 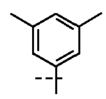 | 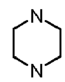 |
| 6 | 6-[(2-Chloro-6-methylphenyl)sulfonyl]-4-piperazin-1-ylquinoline hydrochloride | 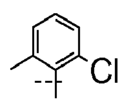 | 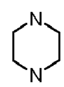 |
| 7 | 6-[(4-Chlorophenyl)sulfonyl]-4-piperazin-1-ylquinoline hydrochloride | 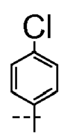 | 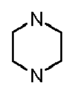 |
| 8 | 6-[(2-Methyl,4-tert-butyl-phenyl)sulfonyl]-4-piperazin-1-ylquinoline hydrochloride | 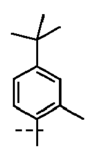 | 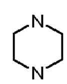 |
| 9 | 6-[(3,4-Dimethylphenyl)sulfonyl]-4-piperazin-1-ylquinoline hydrochloride | 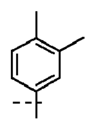 | 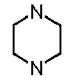 |

(continued)

| EXAMPLE | | $R^6$ | $R^4$ |
|---|---|---|---|
| 10 | 6-[(2,3-Dichlorophenyl)sulfonyl]-4-piperazin-1-ylquinoline hydrochloride | 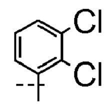 | 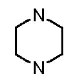 |
| 11 | 6-[(4-tert-Butylphenyl)sulfonyl]-4-piperazin-1-ylquinoline hydrochloride | 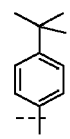 | 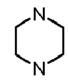 |
| 12 | 6-[(4-Isopropylphenyl)sulfonyl]-4-piperazin-1-ylquinoline hydrochloride | 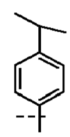 | 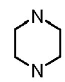 |
| 13 | (4-Piperazin-1-yl-6-{[4-(trifluoromethyl)phenyl]sulfonyl} quinoline hydrochloride | 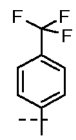 | 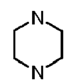 |
| 14 | 6-[(4-tert-Butylphenyl)sulfonyl]-4-(1,4-diazepan-1-yl)quinoline hydrochloride | 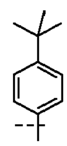 | 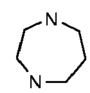 |
| 15 | 4-(1,4-Diazepan-1-yl)-6-[(4-isopropylphenyl)sulfonyl]quinoline hydrochloride | 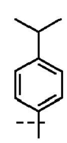 | 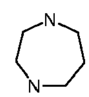 |

**Scheme 1**

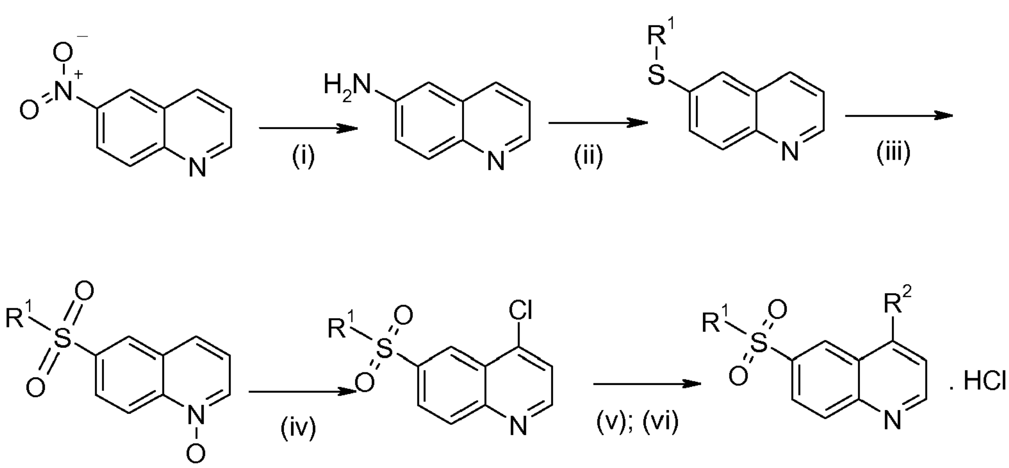


**Legend to Scheme 1**: i) Hydrogen gas, Pd/C, Methanol; ii) Sodium nitrite, Sulphuric acid, diverse thiols ($R^1$-SH), 3h; iii) meta-chloroperoxybenzoic acid (*m*-CPBA), dichloromethane ($CH_2Cl_2$), overnight; iv) phosphorus oxylchloride ($POCl_3$), acetonitrile ($CH_3CN$), 80 ˚C, 2h; v) aliphatic cyclic amines ($R^2$), 80 ˚C, $CH_3CN$; vi) HCl in diethyl ether.

**Methods**

The assigned structures were confirmed by standard spectroscopical methods and elemental analysis and/or high resolution MS.
NMR spectra were obtained on Bruker 500 MHz or JEOL 270 MHz spectrometers at 25˚C, and the chemical shift values are reported as parts per million (δ). MS spectra were acquired on a 2690 Separation Module (Waters) with a Platform LCZ (Micromass). Flash chromatography was performed on Silica gel 60 (Merck) or LiChroprep RP-18 (Merck). HPLC analysis were accomplished on a HP Series 1100, with a GROM-SIL 100 ODS-0 AB column, 4.6x50mm. The HPLC purifications were performed on preparative HPLC/Mass system using YMC Combi prep ODS-AQ column, 56x20 mm, Gilson pumps, Dynamax UV-1 detector and Finnigan Mass detector. The used eluents were $H_2O$ and $CH_3CN$, both with 0.1% TFA. The purity of the compounds was determined by HPLC. Elemental analysis was performed at Structural Chemistry Department, Biovitrum AB, Stockholm. Melting points, when given, were obtained on a Büchi or a Gallenkamp melting point apparatus and are uncorrected.

INTERMEDIATE 1

**Synthesis of 6-Amino-quinoline**

A suspension of 6-nitro-quinoline (8.7 g, 5 mmol), palladium on charcoal (10 %) (0.1 g) in methanol (0.2 L) was hydrogenated at room temperature for 24 with stirring. The catalyst was filtered and the solvent evaporated to yield a yellow solid. Crystallisation from ethyl acetate yielded the pure title compound as a pale yellow solid (3.3 g , 46 %). MS m/z: 145 [M+H+]. $^1$H NMR (270 MHz, $CHCl_3$-d) δ ppm 3.89 (s, 2 H) 6.87 (d, *J*=2.64 Hz, 1 H) 7.14 (dd, *J*=8.97, 2.64 Hz, 1 H) 7.25 (dd, *J*=8.44, 4.22 Hz, 1 H) 7.88 (dd, *J*=7.92, 1.58 Hz, 1 H) 7.90 (d, *J*=8.97 Hz, 1 H) 8.63 (dd, *J*=4.22, 1.58 Hz, 1 H).

INTERMEDIATE 2

**Synthesis of 6-phenylsulfanyl-quinoline**

A solution of sodium nitrite (1 g, 14 mmol) in water (6 mL) was slowly added to a stirred solution of 6-amino-quinoline (1.44 g, 10 mmol) in sulfuric acid (50 %) (8 mL). The temperature was kept below 5 ˚C during the addition. The reaction mixture was poured into a solution of potassium hydroxide (9 g, 16 mmol) and thiophenol (1mL, 9 mmol) in water (30 mL). The reaction mixture was refluxed for 3 h, cooled and extracted with diethyl ether. The insoluble material was eliminated by filtration. During filtration most of the material was trapped in the solid phase. The filtrate was evaporated and the residue was purified by column chromatography ($SiO_2$, ethyl acetate:hexane, 1:2) to yield a colorless oil (100 mg, 4% PS: the low yield is due to the loss of the material during the filtration procedure). MS m/z: 238 [M+H+]. $^1$H NMR (270 MHz, $CD_3Cl$) δ ppm 7.34 (m, 4 H) 7.42 (m, 2 H) 7.57 (dd, *J*=8.97, 2.11 Hz, 1 H) 7.67 (d, *J*=2.11 Hz, 1 H) 7.99 (m, 2 H) 8.84 (dd, *J*=4.22, 1.58 Hz, 1 H).

INTERMEDIATE 3

**Synthesis of 6-benzenesulfonyl-quinoline 1-oxid**

A solution of m-chloroperbenzoic acid (1 g, 5.8 mmol) in DCM (10 mL) was added to a stirred solution of 6-phenylsulfanyl-quinoline (0.25 g, 1 mmol) and $NaHCO_3$ (0.5 g) in DCM (10 mL). The reaction was left stirring over night, washed with water, $NaHCO_3$ solution and evaporated. Trituration of the residue in diethyl ether gave the pure title product as a slightly yellow solid (0.14 g, 30 %). MS m/z: 287 [M+H+].

INTERMEDIATE 4

**Synthesis of 6-benzenesulfonyl-4-chloro-quinoline**

A solution of 6-benzenesulfonyl-quinoline 1-oxid (135 mg, 0.47 mmol) in $POCl_3$ (4 mL) was heated at 90 ˚C for 2 h after which the solution was poured on ice, ammonium hydroxide was added and extraction with DCM. The organic phase was dried ($NaSO_4$), the volatiles were evaporated and the residue was purified by column chromatography ($SiO_2$, ethyl acetate:petroleum ether, 1:1) to yield a white solid (39 mg, 27 %). MS m/z: 305 [M+H+].

EXAMPLE 1

**Synthesis of 6-benzenesulfonyl-4-piperazin-1-yl-quinoline hydrochloride**

A solution of 6-benzenesulfonyl-4-chloro-quinoline (35 mg, 0.11 mmol) and piperazine (0.5 g, 2.5 mmol) in acetonitrile (2 mL) was heated at 80 ˚C over night. The mixture was extracted with toluene and water. The organic phase was purified by chromatography on silica gel eluted with $CHCl_3$ saturated with $NH_3$ (gas). The pure product was dissolved in ethyl acetate and HCl (gas) in diethyl ether was added. The resulting oily residue was dissolved in methanol and ethyl acetate and evaporated to yield a white solid (24 mg, 77%). MS m/z: 354 [M+H+]. $^1$H NMR (270 MHz, $CH_3OH$-$D_4$) δ ppm 3.52 (m, 4 H) 4.13 (m, 4 H) 7.36 (d, *J*=7.18 Hz, 1 H) 7.57 (m, 3 H) 8.01 (m, *J*=12.25, 8.54 Hz, 3 H) 8.28 (d, *J*=8.91 Hz, 1 H) 8.63 (d, *J*=6.68 Hz, 1 H) 8.69 (s, 1 H).

**Scheme 2**

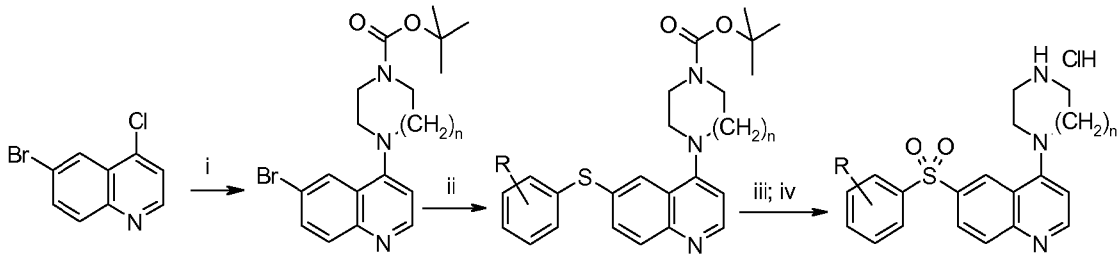


2a, n=0
2b, n=1

**Legend for Scheme 2**: i) Na*t*BuO, $Pd(PPh_3)_4$, n-BuOH, BOC-protected diamines; ii) *tert*-butyl piperazine-1-carboxylate or *tert*-butyl 1,4-diazepane-1-carboxylate, triethylamine or $K_2CO_3$, DMSO, thiols; iv) TFA, $H_2O_2$, NaOH; iv) HCl.

**Method A**

**Preparation of thiol derivatives**

tert-Butyl 4-(6-bromoquinolin-4-yl)-1,4-diazepane-1-carboxylate (0.5 g, 1.23 mmol) was mixed with the thiol (1 equiv.), NaOtBu (2 equiv.), $Pd(PPh_3)_4$ (0.05 equiv.) and n-BuOH (5 mL) in a reaction tube. $N_2$ (g) was flushed through the mixture for 30 minutes. The reaction mixture was heated to 120˚C overnight. The precipitate was filtrated and the reaction mixture concentrated in vacuo. The residue was dissolved in EtOAc and washed with $H_2O$, dried ($MgSO_4$) and evaporated. Purification by flash chromatography using DCM: MeOH 98:2 as eluent afforded the title product that was used in the next step without further purification.

**Method B**

**Oxidation of thiol derivatives to sulphone derivatives**

The appropriate thiophenols derivatives are dissolved in TFA (5 mL) and stirred for 15 minutes at room temperature. $H_2O_2$ (2 mL) was added and the reaction was left stirring overnight. The reaction mixtures are evaporated and the residues are portioned between diethyl ether and water. The layers are separated and the water layer is extracted with diethyl ether and made basic by adding NaOH 1M. Extraction with DCM, drying with $MgSO_4$ and evaporation gives the free bases of the products which are dissolved in MeOH, excess of HCl/ether (2M) was added and the solvent evaporated. The residues are purified on preparative HPLC/MS (Xterra MS C18, 5 μm column) using a 10 to 40% MeCN-water gradient (containing 0.1 % HOAc) over 10 minutes. The pure fractions are pooled and lyophilised. The residues are dissolved in MeOH and treated with excess of HCl/ether (2M). After evaporation of solvent, a solid is obtained and triturated with diethyl ether giving the desired products as HCl-salts.

INTERMEDIATE 5

**tert-Butyl 4-(6-bromoquinolin-4-yl)piperazine-1-carboxylate**

6-Bromo-4-chloroquinoline (5.0 g, 20.6 mmol), *tert*-butyl-1-piperazine (4.1 g, 22 mmol), triethylamine (3 mL, 22 mmol) and DMSO (20 mL) were mixed and heated overnight in an oil bath at 100˚C. The reaction was cooled and diluted with diethyl ether and washed with water (5x), dried ($MgSO_4$) and evaporated. The residue was filtered through a short column of silica (2.5-5 %) MeOH in $CH_2Cl_2$ and evaporated. Yield 8.02 g. (97 %). Brown liquid. HPLC 98 %, $R_T$=3.01 (System A1, 10-97 % MeCN over 3 min). $^1$H NMR (400 MHz, $CDCl_3$) δ ppm 1.52 (s, 9 H) 3.12-3.17 (m, 4 H) 3.69-3.75 (m, 4 H) 6.86 (d, *J*=5.0 Hz, 1 H) 7.72 (dd, *J*=9.0, 2.26 Hz, 1 H) 7.92 (d, *J*=8.8 Hz, 1 H) 8.14 (d, *J*=2.3 Hz, 1 H) 8.73 (d, *J*=5.0 Hz, 1 H). MS (ESI+) for $C_{18}H_{22}BrN_3O_2$ *m*/*z* 392.2 ($M+H^+$)

EXAMPLE 2

**6-[(2-Fluorophenyl)sulfonyl]-4-piperazin-1-ylquinoline hydrochloride**

A total amount of 2.25 mmol, of the appropriate thiophenol was used and the reaction was prolonged with 8 hours. The oxidation step was completed after 24 hours at ambient temperature. Purification by column chromatography on silica gel 10-20 % MeOH in DCM gave the free amine that was additionally purified by preparative HPLC. Yield 15 mg (4 %) Yellow solid. HPLC 95 %, $R_T$=2.33 (System A1, 10-97 % MeCN over 3 min). $^1$H NMR (400 MHz, DMSO-$d_6$) δ ppm 3.30-3.42 (m, 4 H) 3.51-3.62 (m, 4 H) 7.28 (d, *J*=5.27 Hz, 1 H) 7.42 (dd, *J*=10.29, 8.78 Hz, 1 H) 7.52 (t, *J*=7.28 Hz, 1 H) 7.77-7.84 (m, 1 H) 8.04-8.21 (m, 3 H) 8.62 (s, 1 H) 8.87 (d, *J*=5.27 Hz, 1 H) 9.82 (br s, 2 H). MS (ESI+) for $C_{19}H_{18}FN_3O_2S$ *m*/*z* 372.0 ($M+H^+$). HRMS for $C_{19}H_{18}FN_3O_2S$: calcd, 371.1104; found, 371.1102.

EXAMPLE 3

**6-(1-Naphthylsulfonyl)-4-piperazin-1-ylquinoline hydrochloride**

A total amount of 2.25 mmol, of the appropriate thiophenol was used and the reaction was prolonged with 8 hours. The oxidation step was completed after 24 hours at ambient temperature. Purification by column chromatography on silica gel 10-20 % MeOH in DCM gave the free amine that was converted to the HCl-salt. Yield 14 mg (4 %). Grey solid. HPLC 95%, $R_T$=2.54 (System A1, 10-97% MeCN over 3 min). $^1$H NMR (400 MHz, DMSO-$d_6$) δ ppm 3.33 (s, 4 H) 4.06 (s, 4 H) 7.38 (d, *J*=6.78 Hz, 1 H) 7.65 (d, *J*=7.53 Hz, 1 H) 7.69-7.75 (m, 1 H) 7.82 (t, *J*=7.78 Hz, 1 H) 8.12 (d, *J*=8.03 Hz, 1 H) 8.21-8.30 (m, 2 H) 8.38 (d, *J*=8.03 Hz, 1 H) 8.56 (t, *J*=8.53 Hz, 2 H) 8.74-8.79 (m, 2 H) 10.05 (s, 2 H). MS (ESI+) for $C_{23}H_{21}N_3O_2S$ *m*/*z* 404.4 ($M+H^+$) HRMS for $C_{23}H_{21}N_3O_2S$: calcd, 403.1354; found, 403.1365.

EXAMPLE 4

**6-[(3,4-Dichlorophenyl)sulfonyl]-4-piperazin-1-ylquinoline hydrochloride**

A total amount of 2.25 mmol, of the appropriate thiophenol was used and the reaction was prolonged with 8 hours. The oxidation step was completed after 24 hours at ambient temperature. Purification by column chromatography on silica gel 10-20 % MeOH in DCM gave the free amine which was converted to the HCl-salt giving yellow solid. Yield 15 mg (3 %). Yellow solid.. $^1$H NMR (400 MHz, DMSO- $d_6$) δ ppm 3.35-3.41 (m, 4 H) 4.06-4.15 (m, 4 H) 7.40 (d, *J*=6.78 Hz, 1 H) 7.93 (d, *J*=8.53 Hz, 1 H) 8.04 (dd, *J*=8.53, 2.01 Hz, 1 H) 8.27 (d, *J*=9.03 Hz, 1 H) 8.32 (d, *J*=2.01 Hz, 1 H) 8.36-8.42 (m, 1 H) 8.73 (d, *J*=1.51 Hz, 1 H) 8.82 (d, *J*=6.53 Hz, 1 H) 9.86 (s, 2 H). MS (ESI+) for $C_{19}$ $H_{17}$ $Cl_2$ $N_3$ $O_2$ S *m*/*z* 422.2 ($M+H^+$). HRMS for $C_{19}H_{17}ChN_3O_2S$: calcd, 421.0419; found, 421.0422. HPLC 95%, $R_T$=2.69 (System A1, 10-97 % MeCN over 3 min)

EXAMPLE 5

**6-[(3,5-Dimethylphenyl)sulfonyl]-4-piperazin-1-ylquinoline hydrochloride**

The oxidation step was completed after 2 hours at ambient temperature. Purification by column chromatography on silica gel 10-20 % MeOH in DCM gave the free amine which was converted to the HCl-salt giving grey solid. Yield 0.007 g (2 %). Yellow solid. HPLC 90 %, $R_T$=2.57 (System A1, 10-97 % MeCN over 3 min). MS (ESI+) for $C_{21}H_{23}FN_3O_2S$ *m*/*z* 382.2. HRMS for $C_{21}H_{23}FN_3O_2S$: calcd, 381.1511; found, 381.1521.

EXAMPLE 6

**6-[(2-Chloro-6-methylphenyl)sulfonyl]-4-piperazin-1-ylquinoline hydrochloride**

A total amount of 2.25 mmol, of the appropriatethiophenol was used and the reaction was prolonged with 8 hours. Additional $H_2O_2$ (1 mL) was added and the reaction mixture was stirred at 50 ˚C for another 48 hours. Purification by column chromatography on silica gel 10-20 % MeOH in DCM gave the free amine that was converted to the HCl-salt. Yield 33 mg (7.5 %). White solid. $^1$H NMR (400 MHz, DMSO-$d_6$) δ ppm 2.13 (s, 3 H) 2.98 (s, 4 H) 3.72 (s, 4 H) 7.06 (d, *J*=6.78 Hz, 1 H) 7.14 (dd, *J*=11.54, 8.03 Hz, 2 H) 7.23 (t, *J*=7.78 Hz, 1 H) 7.89 (d, *J*=8.78 Hz, 1 H) 7.94-8.00 (m, 1 H) 8.24 (s, 1 H) 8.45 (d, *J*=6.78 Hz, 1 H) 9.68 (s, 2 H). MS (ESI+) for $C_{20}H_{20}ClN_3O_2S$ *m/z* 402.2 ($M+H^+$). HRMS for $C_{20}H_{20}ClN_3O_2S$ : calcd, 401.965; found, 401.967. HPLC 95 %, $R_T$=2.55 (System A1, 10-97 % MeCN over 3 min).

EXAMPLE 7

**6-[(4-Chlorophenyl)sulfonyl]-4-piperazin-1-ylquinoline hydrochloride**

A total amount of 2.25 mmol, of the appropriate thiophenol was used and the reaction was prolonged for another 8 hours. The oxidation step was completed after 24 h at ambient temperature. Purification by column chromatography on silica gel 10-20 % MeOH in DCM gave the free amine that was converted to the HCl-salt. Yield 14 mg (3 %). Yellow solid. HPLC 95 %, $R_T$=2.66 (System A1, 10-97 % MeCN over 3 min). MS (ESI+) for $C_{19}H_{18}CN_3O_2S$ *m/z* 388.2 ($M+H^+$). HRMS for $C_{19}H_{18}ClN_3O_2S$: calcd, 387.0808; found, 387.0821.

EXAMPLE 8

**6-[(2-Methyl,4-tert-butyl-phenyl)sulfonyl]-4-piperazin-1-ylquinoline hydrochloride**

The oxidation step was completed after 2 hours at ambient temperature. Purification by column chromatography on silica gel 10-20 % MeOH in DCM gave the free amine which was converted to the HCl-salt giving gray solid. Yield 17 mg (4 %). HPLC 95 %, $R_T$=2.81 (System A1, 10-97 % MeCN over 3 min). MS (ESI+) for $C_{24}H_{29}N_3O_2S$ *m/z* 424.2 ($M+H^+$). HRMS for $C_{24}H_{29}N_3O_2S$: calcd, 423.1980; found, 423.1969.

EXAMPLE 9

**6-[(3,4-Dimethylphenyl)sulfonyl]-4-piperazin-1-ylquinoline** hydrochloride

The oxidation step was completed after 2 hours at ambient temperature. Purification by column chromatography on silica gel 10-20 % MeOH in DCM gave the free amine which was converted to the HCl-salt. Yield 33 mg (8 %). Yellow solid. $^1$H NMR (400 MHz, DMSO- $d_6$) δ ppm 2.27 (d, *J*=6.27 Hz, 6 H) 3.34 (s, 4 H) 4.12 (s, 4 H) 7.39 (dd, *J*=7.40, 2.13 Hz, 2 H) 7.75 (d, *J*=7.78 Hz, 1 H) 7.81 (s, 1 H) 8.32 (s, 2 H) 8.61 (s, 1 H) 8.78 (d, *J*=6.78 Hz, 1 H) 10.18 (s, 2 H). MS (ESI+) for $C_{21}H_{23}N_3O_2S$ *m/z* 382.2 ($M+H^+$). HRMS for $C_{21}H_{23}N_3O_2S$: calcd, 381.1511; found, 381.1519. HPLC 95 %, $R_T$=2.54 (System A1, 10-97 % MeCN over 3 min).

EXAMPLE 10

**6-[(2,3-Dichlorophenyl)sulfonyl]-4-piperazin-1-ylquinoline hydrochloride**

A total amount of 2.25 mmol, of the appropriate thiophenol was used and the reaction was prolonged for another 8 hours. The oxidation step was completed after 24 hours at ambient temperature. Purification by column chromatography on silica gel 10-20% MeOH in DCM gave the free amine which was converted to the HCl-salt. Yield 15 mg (3 %). Yellow solid. $^1$H NMR (400 MHz, DMSO-$d_6$) δ ppm 3.36 (m, 4 H) 4.10 (m, 4 H) 7.42 (d, *J*=6.78 Hz, 1 H) 7.75 (t, *J*=8.03 Hz, 1 H) 8.07 (d, *J*=8.03 Hz, 1 H) 8.24 (d, *J*=9.04 Hz, 1 H) 8.33 (dd, *J*=13.93, 8.41 Hz, 2 H) 8.70 (s, 1 H) 8.82 (d, *J*=6.78 Hz, 1 H) 10.00 (s, 2 H). MS (ESI+) for $C_{19}H_{17}Cl_2N_3O_2S$ *m/z* 422.2 ($M+H^+$). HRMS for $C_{19}H_{17}Cl_2N_3O_2S$: calcd, 421.0419; found, 421.0408. HPLC 95 %, $R_T$=2.50 (System Al, 10-97 % MeCN over 3 min).

EXAMPLE 11

**6-[(4-tert-Butylphenyl)sulfonyl]-4-piperazin-1-ylquinoline hydrochloride**

tert-Butyl 4-{6-[(4-tert-butylphenyl)thio]quinolin-4-yl}piperazine-1-carboxylate (0.60 g, 1.3 mmol) was dissolved in TFA (12 mL) and stirred for 30 minutes before $H_2O_2$ (0.65 mL, 6.3 mmol) was added. The mixture was stirred for 2 hours and water (5 mL) was added. The mixture was evaporated and the residue was taken up in water and washed with diethyl ether (2x). The aqueous phase was adjusted to pH 10 with 1 N NaOH and the mixture was extracted with $CH_2Cl_2$ (2x), dried ($MgSO_4$) and evaporated. The residue was diluted with $CH_2Cl_2$ and 1.3 mL 2N HCl in diethyl ether was added under vigorous stirring and the mixture was evaporated and washed with diethyl ether (2 x) and dried.Yield: 0.40 g (69 %). Grey solid. HPLC 95 %, $R_T$=2.77 (System A1, 10-97 % MeCN over 3 min). $^1$H NMR (400 MHz, DMSO-$d_6$) δ ppm 1.23 (s, 9 H) 3.38 (s, 4 H) 4.08 (s, 4 H) 7.39 (d, *J*=7.03 Hz, 1 H) 7.65 (d, *J*=8.53 Hz, 2 H) 7.96 (d, *J*=8.53 Hz, 2 H) 8.25 (d, *J*=8.78 Hz, 1 H) 8.30-8.36 (m, 1 H) 8.66 (d, *J*=1.76 Hz, 1 H) 8.81 (d, *J*=7.03 Hz, 1 H) 9.85 (br. s, 2 H), MS (ESI+) for $C_{23}H_{27}N_3O_2S$ *m/z* 410.4 ($M+H^+$).

EXAMPLE 12

**6-[(4-Isopropylphenyl)sulfonyl]-4-piperazin-1-ylquinoline hydrochloride**

4-Isopropylthiophenol (0.152 g, 1.0 mmol) was added dropwise to a suspension of *tert*-butyl 4-(6-bromo-quinolin-4-yl)-piperazine-1-carboxylate (0.2 g, 0.51 mmol), Na-t-butoxide (0.192 g, 2.0 mmol) and $Pd[P(Ph)_3]_4$ (0.030 g, 0.025 mmol) in ethanol (3 mL) at 90˚C and the mixture was stirred for 18 h. The mixture was diluted with THF and filtered through a plug of silica and evaporated. The crude product was dissolved in TFA (5 mL) and stirred for 15 minutes before 30 % $H_2O_2$ (1 mL) was added. The mixture was stirred for 2 hours and evaporated. The residue was dissolved in water and washed with $CH_2Cl_2$ (2x) and 2 N NaOH was added until pH reached 10 and the mixture was extracted with $CH_2Cl_2$ (3x), dried ($MgSO_4$) and evaporated. The crude product was purified by preparative HPLC 5-95 water/acetonitrile collecting on m/z 395.2. After evaporation the free amine was dissolved in $CH_2Cl_2$ and and excess ofHCl in diethyl ether was added and the mixture was evaporated. Yield 0.015 g (7 %). $^1$H NMR (400 MHz, DMSO-$d_6$) δ ppm 1.17 (d, *J*=7.03 Hz, 6 H) 2.90-3.02 (m, 1 H) 3.34-3.42 (m, 4 H) 4.03-4.12 (m, 4 H) 7.39 (d, *J*=6.78 Hz, 1 H) 7.51 (d, *J*=8.28 Hz, 2 H) 7.96 (d, *J*=8.53 Hz, 2 H) 8.26 (d, *J*=9.03 Hz, 1 H) 8.29-8.36 (m, 1 H) 8.66 (s, 1 H) 8.80 (d, *J*=6.78 Hz, 1 H) 9.85-9.97 (m, 2 H). HPLC 95%, $R_T$=2.65 (System A1, 10-97% MeCN over 3 min).

EXAMPLE 13

**4-Piperazin-1-yl-6-{[4-(trifluoromethyl)phenyl]sulfonyl}quinoline hydrochloride**

4-Trifluoromethylthiophenol (0.178 g, 1.0 mmol) was added dropwise to a suspension of *tert*-butyl 4-(6-bromo-quinolin-4-yl)-piperazine-1-carboxylate (0.2 g, 0.51 mmol), Sodium-t-butoxide (0.192 g, 2.0 mmol) and $Pd[P(Ph)_3]_4$ (0.030 g, 0.025 mmol) in ethanol (3 mL) at 90˚C and the mixture was stirred for 18h. The mixture was diluted with THF and filtered through a plug of silica and evaporated. The crude product was dissolved in TFA (5 mL) and stirred for 15 minutes before 30 % $H_2O_2$ (1 mL) was added. The mixture was stirred for 2 hours and evaporated. The residue was dissolved in water and washed with $CH_2Cl_2$ (2x) and 2 N NaOH was added until pH reached 10 and the mixture was extracted with $CH_2Cl_2$ (3x) dried ($MgSO_4$) and evaporated. The crude was purified by preparative HPLC 5-95 water/acetonitrile collecting on m/z 421.1. After evaporation the free amine was dissolved in $CH_2Cl_2$ and excess of HCl in diethyl ether was added and the mixture was evaporated. Yield 0.024 g (10%). $^1$H NMR (400 MHz, DMSO-$d_6$) δ ppm 3.33-3.40 (m, 4 H) 4.13-4.21 (m, 4 H) 7.42 (d, *J*=7.03 Hz, 1 H) 8.02 (d, *J*=8.53 Hz, 2 H) 8.25-8.35 (m, 3 H) 8.37-8.53 (m, 1 H) 8.76 (d, *J*=1.76 Hz, 1 H) 8.80 (d, *J*=7.03 Hz, 1 H) 9.95-10.05 (m, 2 H). Yellow oil. HPLC 95 %, $R_T$=2.66 (System A1, 10-97% MeCN over 3 min).

INTERMEDIATE 6

**tert-Butyl 4-(6-bromoquinolin-4-yl)-1,4-diazepane-1-carboxylate**

6-Bromo-4-chloroquinoline (3.5 g, 14.5 mmol) was reacted with *tert*-butyl 1,4-diazepane-1-carboxylate (3.7 g, 18.8 mmol) and $K_2CO_3$ (4 g, 29 mmol) in DMSO at 100 ˚C overnight. After cooling the mixture was poured into water and extracted with DCM. The organic layer was washed with water, dried ($MgSO_4$) and evaporated. The residue was purified by flash chromatography using a gradient of EtOAc:hexane 1:1 to 2:1 giving 2.1 g (36 %) of yellow oil. $^1$H NMR (400 MHz, $CDCl_3$) δ ppm 1.47 (d, *J*=5.5 Hz, 9 H) 2.08-2.16 (m, 2 H) 3.35-3.44 (m, 4 H) 3.60-3-73 (m, 4 H) 6.87 (d, *J*=5.5

Hz, 1 H) 7.69 (dd, *J*=9.0, 2.0 Hz, 1 H) 7.88 (d, *J*=8.5 Hz, 1 H) 8.16 (s, 1 H) 8.65 (d, *J*=5.0 Hz, 1 H). MS (ESI+) for $C_{19}H_{24}BrN_3O_2$ *m/z* 406.4 $(M+H)^+$. HRMS (EI) calcd for $C_{19}H_{24}BrN_3$: 405.1052, found 405.1045.

INTERMEDIATE 7

**tert-Butyl-4{3-[(4-tert-butylphenyl)thio]quinolin-5-yl}-1,4-diazepane-1-carboxylate**

*(General Method A)*

The compound was prepared from tert-buty14-(6-bromoquinolin-4-yl)-1,4-diazepane-1-carboxylate (0.5 g, 1.23 mmol) and p-*tert*-butylbenzenethiol (0.2 g, 1.23 mmol).Yield: 0.27 g (44 %) of the title compound. HPLC 89 %, $R_T$: 3.76 min (5-99 % MeCN containing 0.1 % TFA over 3 min).

INTERMEDIATE 8

**tert-Butyl-4{3-[(4-isopropylphenyl)thio]quinolin-5-yl}-1,4-diazepane-1-carboxylate**

*(General Method A)*

The compound was prepared from tert-butyl 4-(6-bromoquinolin-4-yl)-1,4-diazepane-1-carboxylate (0.5 g, 1.23 mmol) and 4-isopropylbenzenethiol (0.19 g, 1.23 mmol). Yield: 0.27 g (46 %) of the title compound that was used in the next step without further purification. HPLC 89 %, $R_T$: 3.67 min (5-99 % MeCN containing 0.1 % TFA over 3 min); MS (ESI+) for $C_{28}H_{35}N_3O_2S$ *m/z* 478.2 $(M+H)^+$.

EXAMPLE 14

**6-[(4-tert-Butylphenyl)sulfonyl]-4-(1,4-diazepan-1-yl)quinoline hydrochloride**

***(General Method B)***

The compound was synthesized from tert-butyl-4 {3-[(4-tert-butylphenyl)thio]quinolin-5-yl}-1,4-diazepane-1-carboxylate (0.27 g, 0.55 mmol).
Yield: 20 mg (8 %) of the title compound.; $^1H$ NMR (270 MHz, DMSO-$d_6$) δ ppm 1.25 (s, 9 H) 2.31 (br s, 2 H) 3.25 (br s, 2 H) 3.49 (br s, 2 H) 4.11 br s, 2 H) 4.26 (br s, 2 H) 7.16 (d, *J*=7.1 Hz, 1 H) 7.65 (d, *J*=8.2 Hz, 2 H) 7.94 (d, *J*=8.2 Hz, 2 H) 8.19 (d, *J*=8.7 Hz, 1 H) 8.26 (d, *J*=8.7 1 H) 8.62 (d, *J*=6.3 Hz, 1 H) 8.75 (s, 1 H) 9.65 (br s, 2 H); MS (ESI+) for $C_{24}H_{29}N_3O_2S$ *m/z* 424.2 $(M+H)^+$. HPLC 93%, $R_T$: 2.79 min (5-99 % MeCN over 3 min).

EXAMPLE 15

**4-(1,4-Diazepan-1-yl)-6-[(4-isopropylphenyl)sulfonyl]quinoline hydrochloride**

***(General Method B)***

The compound was prepared from tert-Butyl-4{3-[(4-isopropylphenyl)thio]quinolin-5-yl}-1,4-diazepane-1-carboxylate (0.27 g, 0.57mmol). Yield: 15 mg (6 %) of the title compound.; $^1H$ NMR (270 MHz, DMSO-$d_6$) δ ppm 1.16 (d, *J*=6.9 Hz, 6 H) 2.31 (br s, 2 H) 2.96 (m, 1 H) 3.25 (br s, 2 H) 3.49 (br s, 2 H) 4.11 (br s, 2 H) 4.26 (br s, 2 H) 7.16 (d, *J*=6.9 Hz, 1 H) 7.51 (d, *J*=8.2 Hz, 2 H) 7.94 (d, *J*=8.2 Hz, 2 H) 8.18 (d, *J*=8.7 Hz, 1 H) 8.30 (d, *J*=8.4 Hz, 1 H) 8.62 (d, *J*=6.1 Hz, 1 H) 8.75 (s, 1 H) 9.62 (br s, 2 H); MS (ESI+) for $C_{23}H_{27}N_3O_2S$ *m/z* 410.4 $(M+H)^+$. HPLC 93 %, $R_T$: 2.70 min (5-99 % MeCN over 3 min).

**Table 2**

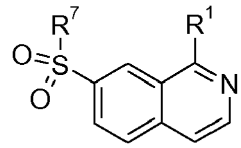

| EXAMPLE | | $R^7$ | $R^1$ |
|---|---|---|---|
| 16 | 7-(2-Chloro-6-methyl-benzenesulfonyl)-1-piperazin-1-yl-isoquinoline hydrochloride | 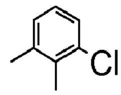 | 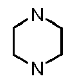 |
| 17 | 7-(2-*t*-Butyl-benzenesulfonyl)-1-piperazin-1-yl-isoquinoline hydrochloride | 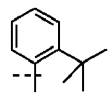 | 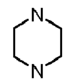 |
| 18 | 7-(3,4-Dichloro-benzenesulfonyl)-1-piperazin-1-yl-isoquinoline hydrochloride | 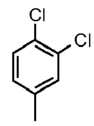 | 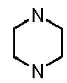 |
| 19 | 7-(3,4-Dimethyl-benzenesulfonyl)-1-piperazin-1-yl-isoquinoline hydrochloride | 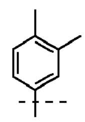 | 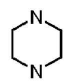 |
| 20 | 7-(2,5-Dimethyl-benzenesulfonyl)-1-piperazin-1-yl-isoquinoline hydrochloride | 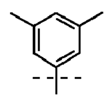 | 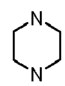 |
| 21 | 7-(*p*-Chloro-benzenesulfonyl)-1-piperazin-1-yl-isoquinoline hydrochloride | 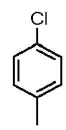 | 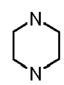 |
| 22 | 7-Benzenesulfonyl-1-[1,4]diazepan-1-yl-isoquinolin hydrochloride | 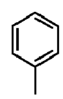 | 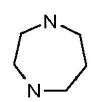 |
| 23 | 7-(4-tert-Butyl-benzenesulfonyl)-1-[1,4]diazepan-1-yl]-isoquinoline, hydrochloride | 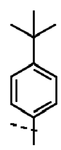 | 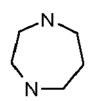 |
| 24 | 7-(2-Chloro-6-methyl-benzenesulfonyl)-1-[1,4]diazepan-1-yl]-isoquinoline hydrochloride | 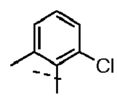 | 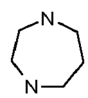 |
| 25 | 7-(3,5-Dimethyl-benzenesulfonyl)-1-[1,4]diazepan-1-yl]-isoquinoline hydrochloride | 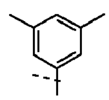 | 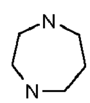 |

(continued)

| EXAMPLE | | $R^7$ | $R^1$ |
|---|---|---|---|
| 26 | 7-(3,4-Dichloro-benzenesulfonyl)-1-[1,4]diazepan-1-yl]-isoquinoline hydrochloride | 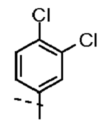 | 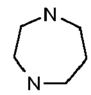 |
| 27 | 7-(4-Chloro-benzenesulfonyl)-1-[1,4]diazepan-1-yl]-isoquinoline hydrochloride | 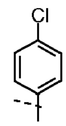 | 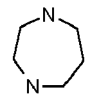 |
| 28 | 7-(3,4-Dimethyl-benzenesulfonyl)-1-[1,4]diazepan-1-yl]-isoquinoline hydrochloride | 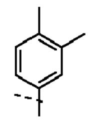 | 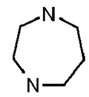 |
| 29 | 7-(2-tert-Butyl-benzenesulfonyl)-1-[1,4]diazepan-1-yl]-isoquinoline hydrochloride | 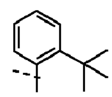 | 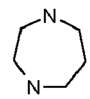 |
| 30 | 7-Benzenesulfonyl-1-piperazin-yl-isoquinoline hydrochloride | 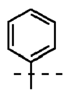 | 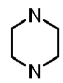 |
| 31 | 7-(4-tert-Butyl-benzenesulfonyl-1-piperazin-yl-isoquinoline hydrochloride | 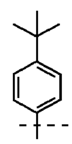 | 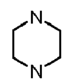 |

**Scheme 3**

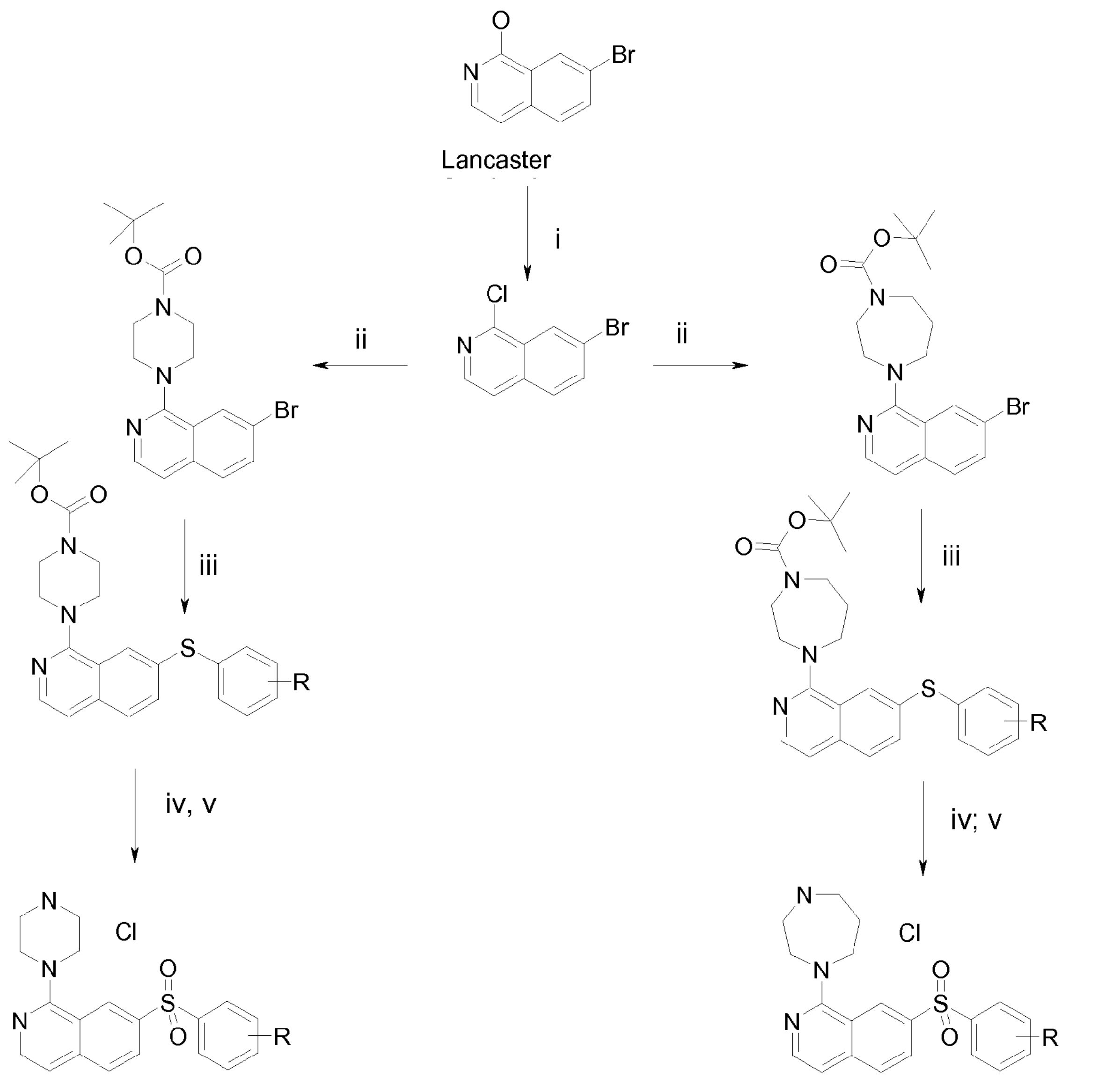


**Legend to Scheme 3:** i) $POCl_3$; ii) $K_2CO_3$, DMF, BOC-diamines; iii) Nat-BuO, thiophenols, $Pd(PPh_3)_4$; n-BuOH; iv) TFA, $H_2O_2$; v) HCl in diethyl ether.

INTERMEDIATE 9

**7-Bromo-1-Chloroisoquinoline**

To phosphorus oxychloride (46.6 mL, 0.5 mol) at room temperature was added, portionwise, 7-bromo-1-hydroxyisoquinoline (11.2 g, 0.05 mol). The mixture was heated to 100 ˚C for 90 min with rapid stirring. On cooling to room temperature, the mixture was poured, cautiously onto ice/water (200 mL). Dropwise addition of aqueous ammonia raised the pH=8 and the resulting precipitate was collected by filtration, washing with cold water. The solid was dried under reduced vacuum at 45 ˚C for 12 h. 13.86 g (115 %) Beige solid isolated. $^1$H NMR (DMSO-$d_6$) $\delta$ 8.4 (s, 1 H), 8.34-8.38 (d, J = 6 Hz, 1 H), 8.03-8.07 (m, 2 H), 7.91-7.96 (d, J = 6 Hz, 1 H); HPLC: 96%; LCMS: 242,244,246.

**Nucleophilic displacement of Chlorine**

INTERMEDIATE 10

**4-(7-Bromo-isoquinoline-1-yl)-piperazine-1-carboxylic acid, *tert*-butyl ester**

To a suspension of 7-bromo-1-chloroisoquinoline (3.14 g, 1 3 mmol) in DMSO (20 mL) at room temperature was added either carboxylic acid tert-butyl (BOC)piperazine (7.23 g, 38.8 mmol) or BOC-homopiperazine (7.77 g, 38.8 mmol) and then potassium carbonate (5.36 g, 39 mmol). The mixture was heated to 110 ˚C for 24 h. On cooling, the mixture was poured onto ice/water (50 mL) and extracted with ethyl acetate (3 x 50 mL). The combined organic phases were washed with water 50 mL) and brine (50 mL). Before drying over anhydrous sodium sulfate. Removal of solvent under reduced pressure gave crude product. Purification was performed by applying the crude material to a plug of silica in a filter funnel and eluting with heptane/ethyl acetate (2:1) and gave 2.73 g (54 %) yellow oil. $^1$H NMR ($CDCl_3$) δ 8.20-8.22 (m, 1 H), 8.13-8.18 (d, *J* = 6 Hz, 1 H), 7.65-7-71 (dd, *J* = 12, 3 Hz, 1 H), 7.59-7.65 (d, *J* = 12 Hz, 1 H), 7.21-7.25 (m, 1 H), 3.64-3.73 (m, 4 H), 7.27-7.36 (m, 4 H), 1.49 (s, 9 H); LCMS: 392,394,395.

INTERMEDIATE 11

**4-(7-Bromo-isoquinoline-1-yl)-[1,4]diazepane-1-carboxylic acid, *tert*-butyl ester**

2.75 g (52 %) yellow oil isolated $^1$H NMR ($CDCl_3$) δ 8.19-8.24 (m, 1 H), 8.06-8.12 (d, *J* = 9 Hz, 1 H), 7.54-7.68 (m, 2 H), 7.11 (m, 1 H), 3.47-3.74 (m, 8 H), 1.98-2.16 (m, 2 H), 1.48 (s, 9 H); LCMS: 406,407,408.

**Palladium-catalysed aryl thiol coupling**

To 7-bromo-1-chloroisoquinoline (1 mmol) in butan-1-ol (20 mL) at room temperature was added sodium *tert*-butoxide (481 mg, 5 mmol), thiol (1.5 mmol) and tetrakis triphenylphosphine palladium (60 mg, catalytic). The mixture was heated to 120 ˚C for 16 h. On cooling to room temperature, the mixture was filtered through silica eluting with THF. Removal of solvent under reduced pressure gave the crude product which was used without further purification in the subsequent step.

INTERMEDIATE 12

**4-[7-(2-Chloro-6-methyl-phenylsulfanyl)-isoquinolin-1-yl]-piperazine-1-carboxylic acid *tert*-butyl ester**

A mixture of 4-(7-bromo-isoquinoline-1-yl)-piperazine-1-carboxylic acid, *tert*-butyl ester (0.5 g, 1.3 mmol), 2-chloro-6-methyl-thiophenol (0.206 g, 1.3 mmol), Na*t*BuO (0.44 g, 4.5 mmol), $Pd(PPh_3)_4$ (74 mg, 0.065 mmol) in nBuOH (10 mL) was heated at 110 ˚C, 3h. The reaction mixture was filtered. The filtrate was concentrated and the residue was dissolved in ethyl acetate. The organic phase was washed with water (50 mL x 3), separated and dried ($MgSO_4$), filtered. The volatiles were evaporated and the residue was purified by flash column chromatography ($SiO_2$, n-heptane: ethyl acetate 8:2) to give 530 mg of the title compound as colourless oil (yield 86.4 %). $^1$H NMR ($CDCl_3$) δ 8.05 (d, 1H), 7.65 (d, 1H), 7.20-7.45 (m, 5H), 7.15 (d, 1H), 3.26-3.40 (m, 4H), 3.10-3.20 (m, 4H), 2.5 (s, 3H), 1.38 (s, 9H).

INTERMEDIATE 13

**4-[7-(2-*t*-butyl-phenylsulfanyl)-isoquinolin-1-yl]-piperazine-1-carboxylic acid *tert*-butyl ester**

A mixture of 4-(7-bromo-isoquinoline-1-yl)-piperazine-1-carboxylic acid, *tert*-butyl ester (0.5 g, 1.3 mmol), 2-t-butyl-thiophenol (0.216 g, 1.3 mmol), Na*t*-BuO (0.44 g, 4.5 mmol), $Pd(PPh_3)_4$ (74 mg, 0.065 mmol) in n-BuOH (10 mL) was heated at 110 ˚C, 3h. The reaction mixture was filtered. The filtrate was concentrated and the residue was dissolved in ethyl acetate. The organic phase was washed with water (50 mL x 3), separated and dried ($MgSO_4$), filtered. The volatiles were evaporated and the residue was purified by flash column chromatography ($SiO_2$, n-heptane: ethyl acetate 8:2) to give 440 mg of the title compound as colorless oil (yield 71 %). $^1$H NMR ($CDCl_3$) δ 8.00-8.10 (m, 2H), 7.15-7.65 (m, 7H), 3.60-3.70 (m, 1H), 3.30-3.45 (m, 4H), 3.05-3.20 (m, 3H), 1.55 (s, 9H), 1.50 (s, 9H).

INTERMEDIATE 14

**4-[7-(3,4-Dichloro-phenylsulfanyl)-isoquinolin-1-yl]-piperazine-1-carboxylic acid *tert*-butyl ester**

A mixture of 4-(7-bromo-isoquinoline-1-yl)-piperazine-1-carboxylic acid, *tert*-butyl ester (0.5 g, 1.3 mmol), 3,4-dichloro-thiophenol (165 uL, 1.3 mmol), Na*t*BuO (0.44 g, 4.5 mmol), $Pd(PPh_3)_4$ (74 mg, 0.065 mmol) in n-BuOH (10 mL) was heated at 110 ˚C, 3h. The reaction mixture was filtered. The filtrate was concentrated and the residue was dissolved in ethyl acetate. The organic phase was washed with water (50 mL x 3), separated and dried ($MgSO_4$), filtered. The volatiles were evaporated and the residue was purified by flash column chromatography ($SiO_2$, n-pentane:ethyl acetate 9.5:0.5→8:2) to give 230 mg of the title compound as colorless oil (yield 36 %). $^1H$ NMR ($CDCl_3$) δ 8.10-8.20 (m, 2H), 7.90 (bs, 1H), 7.65-7.75 (m, 2H), 7.10-7.55 (m, 3H), 3.50-3.65 (m, 4H), 3.20-3.30 (m, 4H), 1.50 (s, 9H).

INTERMEDIATE 15

**4-[7-(3,4-Dimethyl-phenylsulfanyl)-isoquinolin-1-yl]-piperazine-1-carboxylic acid *tert*-butyl ester**

A mixture of 4-(7-bromo-isoquinoline-1-yl)-piperazine-1-carboxylic acid, *tert*-butyl ester (0.5 g, 1.3 mmol), 3,4-dimethyl-thiophenol (175 uL, 1.3 mmol), Na*t*BuO (0.44 g, 4.5 mmol), $Pd(PPh_3)_4$ (74 mg, 0.065 mmol) in n-BuOH (10 mL) was heated at 110 ˚C, 3h. The reaction mixture was filtered. The filtrate was concentrated and the residue was dissolved in ethyl acetate. The organic phase was washed with water (50 mL x 3), separated and dried ($MgSO_4$), filtered. The volatiles were evaporated and the residue was purified by flash column chromatography ($SiO_2$, n-pentane:ethyl acetate 9.5:0.5→8:2) to give 260 mg of the title compound as colorless oil (yield 44 %). $^1H$ NMR ($CDCl_3$) δ 8.00-8.10 (m, 2H), 7.55-7.65 (m, 3H), 7.40-7.50 (m, 1H), 7.10-7.30 (m, 2H), 3.30-3.40 (m, 4H), 3.10-3.20 (m, 4H), 2.30 (s, 3H), 2.25 (s, 3H), 1.50 (s, 9H).

INTERMEDIATE 16

**4-[7-(3,5-Dimethyl-phenylsulfanyl)-isoquinolin-1-yl]-piperazine-1-carboxylic acid *tert*-butyl ester**

A mixture of 4-(7-bromo-isoquinoline-1-yl)-piperazine-1-carboxylic acid, *tert*-butyl ester (0.5 g, 1.3 mmol), 3,5-dimethyl-thiophenol (180 mg, 1.3 mmol), Na*t*BuO (0.44 g, 4.5 mmol), $Pd(PPh_3)_4$ (74 mg, 0.065 mmol) in nBuOH (10 mL) was heated at 110 ˚C, 3h. The reaction mixture was filtered. The filtrate was concentrated and the residue was dissolved in ethyl acetate. The organic phase was washed with water (50 mL x 3), separated and dried ($MgSO_4$), filtered. The volatiles were evaporated and the residue was purified by flash column chromatography ($SiO_2$, n-pentane:ethyl acetate 9.8:0.2→8:2) to give 380 mg of the title compound as colourless oil (yield 65 %). $^1H$ NMR ($CDCl_3$) δ 8.05-8.10 (m, 1H), 7.80-7.85 (m. 1H), 7.60-7.75 (m, 1H), 7.17-7.25 (m, 1H), 7.10 (bs, 2H), 7.00 (bs, 1H), 3.40-3.50 (m, 4H), 3.10-3.20 (m, 4H), 2.25 (bs, 6H), 1.50 (s, 9H).

INTERMEDIATE 17

**4-[7-(*p*-Chloro-phenylsulfanyl)-isoquinolin-1-yl]-piperazine-1-carboxylic acid *tert*-butyl ester**

A mixture of 4-(7-bromo-isoquinoline-1-yl)-piperazine-1-carboxylic acid, *tert*-butyl ester (0.5 g, 1.3 mmol), *p*-chloro-thiophenol (188 mg, 1.3 mmol), Na*t*BuO (0.44 g, 4.5 mmol), $Pd(PPh_3)_4$ (74 mg, 0.065 mmol) in nBuOH (10 mL) was heated at 110 ˚C, 3h. The reaction mixture was filtered. The filtrate was concentrated and the residue was dissolved in ethyl acetate. The organic phase was washed with water (50 mL x 3), separated and dried ($MgSO_4$), filtered. The volatiles were evaporated and the residue was purified by flash column chromatography ($SiO_2$, n-pentane:ethyl acetate 9.5:0.5 → 8:2) to give 300 mg of the title compound as colourless oil (yield 50 %). $^1H$ NMR ($CDCl_3$) δ 8.05-8.15 (m, 2H), 7.60-7.70 (m, 2H), 7.40-7.50 (m, 2H), 7.15-7.30 (m, 3H), 3.45-3.55 (m, 4H), 3.10-3.15 (m, 4H), 1.50 (s, 9H).

INTERMEDIATE 18

**4-(7-Phenylsulfanyl-isoquinoline-1-yl)-piperazine-1-carboxylic acid, *tert*-butyl ester**

LCMS: 422,423.

INTERMEDIATE 19

**4-[7-(4-*tert*-Butyl-phenylsulfanyl)-isoquinoline-1-yl]-piperazine-1-carboxylic acid, *tert*-butyl ester**

LCMS: 478,479.

INTERMEDIATE 20

**4-(7-Phenylsulfanyl-isoquinoline-1-yl)-[1,4]diazepane-1-carboxylic acid, *tert*-butyl ester**

MS: 368,369,370.

INTERMEDIATE 21

**4-[7-(4-*tert*-Butyl-phenylsulfanyl)-isoquinoline-1-yl]-[1,4]diazepane-1-carboxylic acid, *tert-butyl* ester**

MS: 424,425,426.

INTERMEDIATE 22

**4-[7-(2-Chloro-6-methyl-phenylsulfanyl)-isoquinoline-1-yl]-[1,4] diazepane-1-carboxylic acid, *tert*-butyl ester**

MS: 416,417,418.

INTERMEDIATE 23

**4-[7-(3,4-Dimethyl-phenylsulfanyl)-isoquinoline-1-yl]-[1,4] diazepane-1-carboxylic acid, *tert*-butyl ester**

MS: 396,397,398.

INTERMEDIATE 24

**4-[7-(3,4-Dichloro-phenylsulfanyl)-isoquinoline-1-yl]-[1,4]diazepane-1-carboxylic acid, *tert*-butyl ester**

MS: 436,437438.

INTERMEDIATE 25

**4-[7-(4-Chloro-phenylsulfanyl)-isoquinoline-1-yl]-[1,4]diazepane-1-carboxylic acid, *tert*-butyl ester**

MS: 402,404.

INTERMEDIATE 26

**4-[7-(3,4-Dimethyl-phenylsulfanyl)-isoquinoline-1-yl]-[1,4] diazepane-1-carboxylic acid, *tert*-butyl ester**

LCMS: 464,465,466.

INTERMEDIATE 27

**4-[7-(2-*tert*-Butyl-phenylsulfanyl)-isoquinoline-1-yl]-[1,4]diazepane-1-carboxylic acid, *tert*-butyl ester**

LCMS: 492,493,494.

**BOC deprotection and oxidation of thiols to sulphone derivatives**

Each thiol (0.2-1.14 mmol) was dissolved in trifluoroacetic acid (1.5 mL) at 0 ˚C and stirred for 15 mins at this temperature. To this was added 33% aqueous hydrogen peroxide solution (5-100 mL). The resulting mixture was stirred

at room temperature for 90 min and then treated with sodium hydroxide solution (1M, 25 mL). Extraction of this mixture with ethyl acetate (3 x 50 mL) was followed by the washing of the combined organic layers with brine (50mL). The organic extracts were dried over anhydrous sodium sulfate and then the solvent was removed under reduced pressure. The crude product was purified by preparative LCMS. Treatment of the purified material with HCl/Ether (1M, 1 mL) gave the final product as a white solid.

EXAMPLE 16

**7-(2-Chloro-6-methyl-benzenesulfonyl)-1-piperazin-1-yl-isoquinoline hydrochloride**

A mixture of 4-[7-(2-chloro-6-methyl-phenylsulfanyl)-isoquinolin-1-yl]-piperazine-1-carboxylic acid *tert*-butyl ester (160 mg, 0.340 mmol), $H_2O_2$ (30% in water, 200 uL), trifluoroacetic acid (2 mL) was heated at 50 ˚C, 2h. A water solution of NaOH (1N) was added (pH = 14), ethyl acetate was added and the organic phase was separated, dried ($MgSO_4$), filtered. The filtrated was concentrated and the residue was purified by flash column chromatography ($SiO_2$, dichloromethane:methanol 8:2) to lead to 77 mg of the product compound as free base (yield 56 %). The free base was converted into hydrochloride by treatment with HCl in diethyl ether. $^1$H NMR ($CH_3OH$-$d_4$) δ 8.88 (bs, 1H), 8.05-8.20 (m. 3H), 7.65 (d, 1H), 7.35-7.55 (m, 3H), 3.85-3.95 (m, 4H), 3.00-3.15 (m, 4H), 2.95 (s, 3H).

EXAMPLE 17

**7-(2-*t*-Butyl-benzenesulfonyl)-1-piperazin-1-yl-isoquinoline hydrochloride**

A mixture of 4-[7-(2-*t*butyl-phenylsulfanyl)-isoquinolin-1-yl]-piperazine-1-carboxylic acid *tert*-butyl ester (273 mg, 0.571 mmol), $H_2O_2$ (30% in water, 1 mL), trifluoroacetic acid (3 mL) was heated at 50 ˚C, 2h. The reaction was continued overnight at 35 ˚C. A water solution of NaOH (1N) was added (pH = 14), ethyl acetate was added and the organic phase was separated, dried ($MgSO_4$), filtered. The filtrated was concentrated and the residue was purified by flash column chromatography ($SiO_2$, dichloromethane:methanol 8:2) to lead to 50 mg of the title compound as free base (yield 56 %). The free base was converted into hydrochloride by treatment with HCl in diethyl ether. $^1$H NMR ($CH_3OH$-$d_4$) δ 8.55 (d, 1H), 8.25 (d, 1H), 7.95-8.10 (m, 3H), 7.55 (d, 1H), 7.55-7.65 (m, 2H), 7.4 (d, 1H), 3.60-3.75 (m, 4H), 3.40-3.50 (m, 4H), 1.55 (s, 9H).

EXAMPLE 18

**7-(3,4-Dichloro-benzenesulfonyl)-1-piperazin-1-yl-isoquinoline hydrochloride**

A mixture of 4-[7-(3,4-dichloro-phenylsulfanyl)-isoquinolin-1-yl]-piperazine-1-carboxylic acid *tert*-butyl ester (230 mg, 0.47 mmol), $H_2O_2$ (30% in water, 0.5 mL), trifluoroacetic acid (1.5 mL) was heated at 50 ˚C, 2h. The reaction was continued overnight at 35 ˚C. A water solution of NaOH (1N) was added (pH = 14), ethyl acetate was added and the organic phase was separated, dried ($MgSO_4$), filtered. The filtrated was concentrated and the residue was purified by flash column chromatography ($SiO_2$, dichloromethane:methanol 9:1) The free base was converted into hydrochloride by treatment with HCl in diethyl ether to obtained 45 mg of the title compound. $^1$H NMR ($CH_3OH$-$d_4$) δ 8.75-8.85 (m, 1H), 8.10-8.30 (m, 4H), 7.90-8.00 (m, 1H), 7.75-7.85 (m, 1H), 7.50-7.60 (m, 1H), 3.85-3.90 (m, 4H), 3.50-3.70 (m, 4H).

EXAMPLE 19

**7-(3,4-Dimethyl-benzenesulfonyl)-1-piperazin-1-yl-isoquinoline hydrochloride**

A mixture of 4-[7-(3,4-dimethyl-phenylsulfanyl)-isoquinolin-1-yl]-piperazine-1-carboxylic acid *tert*-butyl ester (260 mg, 0.58 mmol), $H_2O_2$ (30% in water, 0.5 mL), trifluoroacetic acid (1.5 mL) was heated at 50 ˚C, 2h. The reaction was continued overnight at 35 ˚C. A water solution of NaOH (1N) was added (pH = 14), ethyl acetate was added and the organic phase was separated, dried ($MgSO_4$), filtered. The filtrated was concentrated and the residue was purified by flash column chromatography ($SiO_2$, dichloromethane:methanol 9:1) The free base was converted into hydrochloride by treatment with HCl in diethyl ether to obtained 20 mg of the title compound. $^1$H NMR ($CH_3OH$-$d_4$) δ 8.75-8.80 (m, 1H), 8.10-8.25 (m, 3H), 7.70-7.85 (m, 2H), 7.60-7.70 (m, 1H), 7.35-7.40 (m, 1H), 3.90-4.00 (m, 4H), 3.55-3.65 (m, 4H), 2.35 (bs, 6H).

EXAMPLE 20

**7-(2,5-Dimethyl-benzenesulfonyl)-1-piperazin-1-yl-isoquinoline hydrochloride**

A mixture of 4-[7-(2,5-dimethyl-phenylsulfanyl)-isoquinolin-1-yl]-piperazine-1-carboxylic acid *tert*-butyl ester (380 mg, 0.846 mmol), $H_2O_2$ (30% in water, 0.5 mL), trifluoroacetic acid (3 mL) was heated at 50 ˚C, 2h. The reaction was continued overnight at 35 ˚C. A water solution of NaOH (1N) was added (pH = 14), ethyl acetate was added and the organic phase was separated, dried ($MgSO_4$), filtered. The filtrated was concentrated and the residue was purified by flash column chromatography ($SiO_2$, dichloromethane:methanol 9.8:0.2→9.5:0.5) The free base was converted into hydrochloride by treatment with HCl in diethyl ether to obtained 120 mg of the title compound. $^1$H NMR ($CH_3OH$-*d*$_4$) δ 8.75-8.80 (m, 1H), 8.25-8.30 (m, 1H), 8.05-8.20 (m, 2H), 7.60-7.70 (m, 3H), 7.30-7.35 (m, 1H), 4.00-4.10 (m, 4H), 3.60-3.70 (m, 4H), 2.30-1.35 (bs, 6H).

EXAMPLE 21

**7-(*p*-Chloro-benzenesulfonyl)-1-piperazin-1-yl-isoquinoline hydrochloride**

A mixture of 4-[7-(*p*-chloro-phenylsulfanyl)-isoquinolin-1-yl]-piperazine-1-carboxylic acid *tert*-butyl ester (297 mg, 0.65 mmol), $H_2O_2$ (30% in water, 0.5 mL), trifluoroacetic acid (3 mL) was heated at 50 ˚C, 2h. The reaction was continued overnight at 35 ˚C. A water solution of NaOH (1N) was added (pH = 14), ethyl acetate was added and the organic phase was separated, dried ($MgSO_4$), filtered. The filtrated was concentrated and the residue was purified by flash column chromatography ($SiO_2$, dichloromethane:methanol 9.5:0.5→9.0:1.0) The free base was converted into hydrochloride by treatment with HCl in diethyl ether to obtained 70 mg of the title compound. $^1$H NMR ($CH_3OH$-*d*$_4$) δ 8.75-8.85 (m, 2H), 8.10-8.25 (m, 3H), 8.00-8.08 (m, 2H), 7.60-7.68 (m, 3H), 3.85-3.95 (m, 4H), 3.55-3.65 (m, 4H).

EXAMPLE 22

**7-Benzenesulfonyl-1-[1,4]diazepan-1-yl-isoquinoline hydrochloride**

30 mg. $^1$H NMR (DMSO-*d*$_6$) δ 9.3 (s, 1 H), 8.58 (s, 1 H), 8.26-8.30 (d, *J* = 9 Hz, 1 H), 8.1-8.13 (m, 2 H), 8.01-8.06 (d, *J* = 6 Hz, 1 H), 7.6-7.76 (m, 3 H), 7.53-7.58 (d, *J* = 6 Hz, 1 H), 3.70-3.90 (m, 4H) 3.58-3.66 (m, 2 H), 3.29-3.40 (m, 2H); LCMS: 368,369 HPLC: 98 %.

EXAMPLE 23

**7-(4-*tert*-Butyl-benzenesulfonyl)-1-[1,4]diazepan-1-yl]-isoquinoline hydrochloride**

Isolated 69 mg. $^1$H NMR (DMSO-*d*$_6$) δ 9.2 (s, 1 H), 8.65 (s, 1 H), 8.09-8.15 (d, *J* = 6 Hz, 1 H), 8.03-8.08 (d, *J* = 15 Hz, 1 H), 7.89-7.96 (d, *J* = 9 Hz, 2 H), 7.60-7.67 (d, *J* = 9 Hz, 2 H), 7.33-7.38 (d, *J* = 9 Hz, 1 H), 4.01-4.09 (m, 2 H), 3.83-3.91 (m, 2 H), 3.43-3.52 (m, 2 H), 3.23-3.33 (m, 2 H), 1.25 (s, 9 H); LCMS: 424,425, HPLC: 97 %.

EXAMPLE 24

**7-(2-Chloro-6-methyl-benzenesulfonyl)-1-[1,4]diazepan-1-yl]-isoquinoline hydrochloride**

Isolated 27 mg $^1$H NMR (DMSO-*d*$_6$) δ 8.81 (s, 1 H), 8.28 (m, 1 H), 8.10-8.18 (d, *J* = 6 Hz, 1 H), 7.94-8.08 (m, 2 H), 7.45-7.62 (m, 3 H), 7.36-7.42 (d, *J* = 6 Hz, 1 H), 3.75-3.86 (m, 2 H), 3.41-3.51 (m, 2 H), 3.18-3.32 (m, 2 H), 2.86 (s, 3 H), 2.14-2.19(m 2 H); LCMS: 416,418 HPLC: 98 %.

EXAMPLE 25

**7-(3,5-Dimethyl-benzenesulfonyl)-1-[1,4]diazepan-1-yl]-isoquinoline hydrochloride**

Isolated 62 mg. $^1$H NMR (DMSO-*d*$_6$) δ 9.35 (s, 1 H), 8.67 (m, 1 H), 8.00-8.18 (m, 3 H), 7.58-7.69 (m, 2 H), 7.45-7.41 (d, *J* = 6 Hz, 1 H), 7.30-7.35 (m, 1 H), 4.06-4.14 (m, 2 H), 3.86-3.97 (m, 2 H), 3.42-3.52 (m, 2 H), 3.23-3.31 (m, 2 H), 2.33 (s, 6 H) 2.23-2.25 (m 2 H); LCMS: 436,438, HPLC: 95 %.

EXAMPLE 26

**7-(3,4-Dichloro-benzenesulfonyl)-1-[1,4]diazepan-1-yl]-isoquinoline, hydrochloride**

Isolated 11 mg. $^1$H NMR ($CD_3OD$) δ 8.88 (m, 1 H), 8.23-8.29 (d, *J* = 12 Hz, 1 H), 8.13-8.16 (d, *J* = 3 Hz, 1 H), 8.04-8.10 (d, *J* = 9 Hz, 1 H), 7.88-7.94 (d, *J* = 9 Hz, 1 H), 7.79-7.84 (d, *J* = 6 Hz, 1 H), 7.67-7.73 (d, *J* = 9 Hz, 1 H), 7.42-7.46 (d, *J* = 6 Hz, 1 H), 4.28-4.35 (m, 2 H), 4.09-4.16 (m, 2 H), 3.69-3.75 (m, 2 H), 2.33-2.46 (m, 2 H); LCMS: 368,369; HPLC: 97 %.

EXAMPLE 27

**7-(4-Chloro-benzenesulfonyl)-1-[1,4]diazepan-1-yl]-isoquinoline, hydrochloride**

Isolated 41 mg. $^1$H NMR (DMSO-$d_6$) δ 9.27 (s, 1 H), 8.68 (m, 1 H), 7.99-8.17 (m, 5 H), 7.66-7.75 (d, *J* = 9 Hz, 2 H), 7.33-7.39 (d, *J* = 6 Hz, 1 H), 4.03-4.11 (m, 2 H), 3,83-3.93 (m, 2 H), 3.43-3.53 (m, 2 H), 3.23-3.32 (m, 2 H), 2.19-2.30 (m, 2 H); LCMS: 402,404; HPLC: 98 %.

EXAMPLE 28

**7-(3,4-Dimethyl-benzenesulfonyl)-1-[1,4]diazepan-1-yl]-isoquinoline, hydrochloride**

Isolated 10 mg. $^1$H NMR (DMSO-*$d_6$*) δ 9.41 (s, 1 H), 8.67 (s, 1 H), 8.00-8.16 (m, 3 H), 7.76-7.82 (m, 1 H), 7.68-7.77 (d, *J* = 9 Hz, 1 H), 7.32-7.42 (d, *J* = 9 Hz, 2 H), 3.99-4.40 (m, 4 H), 3.49 (m, 2 H), 3.33 (m, 2 H), 2.29 (s, 3 H), 2.25 (s, 3 H); LCMS: 396,397; HPLC: 92 %.

EXAMPLE 29

**7-(2-*tert*-Butyl-benzenesulfonyl)-1-[1,4]diazepan-1-yl]-isoquinoline, hydrochloride**

Isolated 5 mg. $^1$H NMR (DMSO-$d_6$) δ 9.27 (s, 1 H), 8.52 (s, 1 H), 8.06-8.16 (m, 2 H), 7.97-7.97 (m, 2 H), 7.72-7.78 (m, 1 H), 7.61-7.70 (m, 1 H), 7.40-7.45 (d, J = 9 Hz, 2 H), 3.69-3.99 (m, 4 H), 3.43 (s, 2 H), 3.25 (s, 2 H), 2.05-2.26(m, 2 H);1.52 (s, 9 H); LCMS: 424,425; HPLC: 90 %.

EXAMPLE 30

**7-Benzenesulfonyl-1-piperazin-yl-isoquinoline, hydrochloride**

Isolated 10 mg. $^1$H NMR (DMSO-*$d_6$*) δ 9.04 (s, 1 H), 8.65 (s, 1 H), 8.12-8.16 (d, *J* = 6 Hz, 1 H), 7.98-8.05 (m, 5 H), 7.58-7.72 (m, 2 H), 7.32-7.36 (d, *J* = 6 Hz, 1 H), 3.98-4.04 (m, 4 H), 3.80-3.86 (m, 4 H); LCMS: 354,355; HPLC: 98 %.

EXAMPLE 31

**7-(4-*tert*-Butyl-benzenesulfonyl-1-piperazin-yl-isoquinoline, hydrochloride**

Isolated 10 mg. $^1$H NMR (DMSO-*$d_6$*) δ 9.33 (s, 1 H), 8.57 (s, 1 H), 8.24-8.29 (d, *J* = 9 Hz, 1 H), 8.11 (m, 2 H), 7.91-7.97 (d, *J* = 9 Hz, 2 H), 7.60-7.66 (d, *J* = 12 Hz, 2 H), 7.52-7.57 (d, *J* = 6 Hz, 1 H), 3.59-3.68 (m, 4 H), 3.29-3.40 (m, 4 H), 1.24 (s, 9 H); LCMS: 410,411 HPLC: 90 %.

BIOLOGICAL TESTS

The ability of a compound according to the invention to bind a 5-$HT_6$ receptor, and to be pharmaceutically useful, can be determined using *in vivo* and *in vitro* assays known in the art.

*(a) 5-$HT_6$ binding Assay*

Binding affinity experiment for the 5-$HT_6$ receptor are performed in HEK293 cells transfected with 5-$HT_6$ receptor using (3H)-LSD as labeled ligand according to the general method as described by Boess F.G et al. Neuropharmacology vol. 36(4/5) 713-720, 1997.

## Materials

Cell culture

The HEK-293 cell line transfected with the 5-$HT_6$ receptor was cultured in Dulbeccos Modified Eagles Medium containing 5 % dialyzed foetal bovine serum, (Gibco BRL 10106-169), 0.5 mM sodium pyruvate and 400 μg/ml Geneticin (G-418) (Gibco BRL10131-019). The cells were passaged 1:10, twice a week.

Chemicals

The radio ligand [$^3$H] LSD 60-240 Ci/mmol, obtained from Amersham Pharmacia Biotech, (Buckinghamshire, England) was in ethanol and stored at -20°C. The unlabelled ligands, representing different selectivity profiles, are presented in Table 1. The compounds were dissolved in 100% DMSO and diluted with binding buffer.

Disposable

Compounds were diluted in Costar 96 well V-bottom polypropylene plates (Coming Inc. Costar, NY, USA). Samples were incubated in Packard Optiplate (Packard Instruments B.V., Groningen, The Netherlands). The total amount of added radio ligand was measured in Packard 24-well Barex plates (Packard Instruments B.V., Groningen, The Netherlands) in the presence of Microscint™ 20 scintillation fluid (Packard Bioscience, Meriden, CT, USA).

Buffer

The binding buffer consisted of 20 mM HEPES, 150 mM NaCl, 10 mM $MgCl_2$, and 1 mM, EDTA, pH 7.4.

## Methods

Membrane preparation

Cells were grown to approximately 90% confluence on 24.5 x 24.5 NUNC culture dishes. The medium was aspirated, and after rinsing with ice-cold PBS, the cells were scraped off using 25 ml Tris buffer (50 mM Tris-HCl, 1 mM EDTA, 1 mM EGTA, pH 7.4) and a window scraper. The cells were then broken with a Polytron homogeniser, and remaining particulate matter was removed by low-speed centrifugation, 1000x g for 5 min. Finally, the membranes were collected by high-speed centrifugation (20 000x g), suspended in binding buffer, and frozen in aliquots at -70°C.

Radioligand binding

Frozen cell membranes were thawed, immediately rehomogenized with a Polytron homogenizer, and coupled to SPA wheat germ agglutinin beads (Amersham Life Sciences, Cardiff, England) for 30 min under continuous shaking of the tubes. After coupling, the beads were centrifuged for 10 minutes at 1000 g, and subsequently suspended in 20 ml of binding buffer per 96-well plate The binding reaction was then initiated by adding radio ligand and test compounds to the bead-membrane suspension. Following incubation at room temperature, the assay plates were subjected to scintillation counting. The original SPA method was followed except for that membranes were prepared from HEK293 cells expressing the human 5-$HT_6$ receptor instead of from HeLa cells (Dinh DM, Zaworski PG, Gill GS, Schlachter SK, Lawson CF, Smith MW. Validation of human 5-$HT_6$ receptors expressed in HeLa cell membranes: saturation binding studies, pharmacological profiles of standard CNS agents and SPA development. The Upjohn Company Technical Report 7295-95-064 1995;27 December). The specific binding of [$^3$H]LSD was saturable, while the non-specific binding increased linearly with the concentration of added radio ligand. [$^3$H] LSD bound with high affinity to 5-$HT_6$ receptors. The $K_d$ value was estimated to 2.6$\pm$ 0.2 nM based on four separate experiments.

The total binding at 3 nM of [$^3$H] LSD, the radio ligand concentration used in the competition experiments, was typically 6000 dpm, and the specific binding more than 70%. 5-HT caused a concentration dependent inhibition of [$^3$H] LSD binding with an over all average Ki value of 236 nM when tested against two different membrane preparations. The inter assay variability over three experiments showed a CV of 10% with an average $K_i$ values of 173 nM (SD 30) and a Hill coefficient of 0.94 (SD 0.09). The intra assay variation was 3% (n=4). Ki values for a limited set of reference compounds with reported binding affinities at 5-$HT_6$ receptor are presented in Table 7. All unlabelled ligands displaced the specific binding of [$^3$H] LSD in a concentration-dependent manner, albeit at different potencies. The rank order of potency for the compounds was methiothepin (2 nM) >mianserin (190 nM) $\approx$ 5-HT (236 nM) >methysergide (482 nM) >mesulergide (1970 nM).

**Protein determination**

Protein concentrations were determined with BioRad Protein Assay (Bradford MM. A rapid and sensitive method for the quantitation of microgram quantities of protein utilizing the principle of protein-dye binding. Anal Biochem 1976; 72:248-54). Bovine serum albumin was used as standard.

**Scintillation counting**

The radioactivity was determined in a Packard TopCount™ scintillation counter (Packard Instruments, Meriden, CT, USA) at a counting efficiency of approximately 20 %. The counting efficiency was determined in separate sets of experiments.

**Saturation experiments**

At least 6 concentrations in duplicates of radioligand (0.1-20 nM of [$^3$H] LSD) were used in saturation experiments. The specific binding was calculated as the difference between total binding and non-specific binding, which was determined as the binding of radioligand in the presence of 5 μM lisuride. $B_{max}$ and the dissociation constant, $K_d$, were determined from the non-linear regression analysis using equation 1. $L_u$ is the unbound concentration of radio ligand, and is *y* is the amount bound.

$$y = \frac{B_{max} \cdot Lu}{Lu + Kd} \qquad \text{(equation 1)}$$

**Competition experiments**

Total- and non-specific binding of radioligand was defined in eight replicates of each. Samples containing test compound were run in duplicate at 11 concentrations. Incubations were carried out at room temperature for 3 hours. The $IC_{50}$ value, i.e. the concentration of test compound that inhibited 50% of the specific binding of radioligand, was determined with non linear regression analysis and the $K_i$ value was calculated using the method of [Cheng Y.C. Biochem. Pharmacol. 22, 3099-3108, 19735] equation 2.

$$Ki = \frac{IC_{50}}{1 + \frac{L}{K_d}} \qquad \text{(equation 2)}$$

L = concentration of radioligand
$K_d$ = Affinity of radio ligand

*(b) 5-HT$_6$ Intrinsic Activity Assay*

Antagonists to the 5-$HT_6$ receptor were characterized by measuring inhibition of 5-HT induced increase in cAMP in HEK 293 cells expressing the human 5-$HT_6$ receptor (see Boess et al. (1997) Neuropharmacology 36: 713-720). Briefly, HEK293/5-$HT_6$ cells were seeded in polylysine coated 96-well plates at a density of 25,000 / well and grown in DMEM (Dulbecco's Modified Eagle Medium) (without phenol-red) containing 5% dialyzed Foetal Bovine Serum for 48 h at 37°C in a 5% $CO_2$ incubator. The medium was then aspirated and replaced by 0.1 ml assay medium (Hanks Balance Salt Solution containing 20 mM HEPES, 1.5 mM isobutylmethylxanthine and 1 mg/ml bovine serum albumin). After addition of test substances, 50 μl dissolved in assay medium, the cells were incubated for 10 min at 37°C in a 5% $CO_2$ incubator. The medium was again aspirated and the cAMP content was determined using a radioactive cAMP kit (Amersham Pharmacia Biotech, BIOTRAK RPA559). The potency of antagonists was quantified by determining the concentration that caused 50% inhibition of 5-HT (at [5-HT]= 8 times $EC_{50}$) evoked increase in cAMP, using the formula $IC_{50,corr}=IC_{50}/(1+[5HT]/EC_{50})$.

The compounds in accordance with the invention have a selective affinity to 5-$HT_6$ receptors with Ki and $IC_{50,corr}$ values between 0.5 nM and 5 μM or display a % inhibition of [$^3$H] LSD ≥ 20 % at 50 nM and are antagonists, agonist or

partial agonist at 5-$HT_6$ . The compounds show good selectivity over 5-$HT_{1a}$, 5-$HT_{2a}$, 5-$HT_{2a}$, 5-$HT_{2b}$, 5-$HT_{2c}$.

*(c) In vivo assay of reduction of food intake*

For a review on serotonin and food intake, see Blundell, J.E. and Halford, J.C.G. (1998) Serotonin and Appetite Regulation. Implications for the Pharmacological Treatment of Obesity. CNS Drugs 9:473-495.

Obese (ob/ob) mouse is selected as the primary animal model for screening as this mutant mouse consumes high amounts of food resulting in a high signal to noise ratio. To further substantiate and compare efficacy data, the effect of the compounds on food consumption is also studied in wild type (C57BL/6J) mice. The amount of food consumed during 15 hours of infusion of compounds is recorded.

Male mice (obese C57BL/6JBom-$Lep^{ob}$ and lean wild-type C57B1/6JBom; Bomholtsgaard, Denmark) 8-9 weeks with an average body weight of 50 g (obese) and 25 g (lean) are used in all the studies. The animals are housed singly in cages at 23±1°C, 40-60 % humidity and have free access to water and standard laboratory chow. The 12/12-h light/dark cycle is set to lights off at 5 p.m. The animals are conditioned for at least one week before start of study.

The test compounds are dissolved in solvents suitable for each specific compound such as cyclodextrin, cyclodextrin/methane sulfonic acid, polyethylene glycol/methane sulfonic acid, saline. Fresh solutions are made for each study. Doses of 30, 50 and 100 mg $kg^{-1}day^{-1}$ are used. The purity of the test compounds is of analytical grade.

The animals are weighed at the start of the study and randomized based on body weight. Alzet osmotic minipumps (Model 2001D; infusion rate 8 μl/h) are used and loaded essentially as recommended by the Alzet technical information manual (Alza Scientific Products, 1997; Theeuwes, F. and Yam, S.I. Ann. Biomed. Eng. 4(4). 343-353, 1976). Continuous subcutaneous infusion with 24 hours duration is used. The minipumps are either filled with different concentrations of test compounds dissolved in vehicle or with only vehicle solution and maintained in vehicle pre-warmed to 37°C (approx. 1h). The minipumps are implanted subcutaneously in the neck/back region under short acting anesthesia (metofane/enflurane). This surgical procedure lasts approximately 5 min. It takes about 3 h to reach steady state delivery of the compound.

The weight of the food pellets are measured at 5 p.m. and at 8 p. m. for two days before (baseline) and one day after the implantation of the osmotic minipumps. The weigh-in is performed with a computer assisted Mettler Toledo PR 5002 balance. Occasional spillage is corrected for. At the end of the study the animals are killed by neck dislocation and trunk blood sampled for later analysis of plasma drug concentrations.

The plasma sample proteins are precipitated with methanol, centrifuged and the supernatant is transferred to HPLC vials and injected into the liquid chromatography /mass spectrometric system. The mass spectrometer is set for electrospray positive ion mode and Multiple Reaction Monitoring. A linear regression analysis of the standards forced through the origin is used to calculate the concentrations of the unknown samples.

Food consumption for 15 hours is measured for the three consecutive days and the percentage of basal level values is derived for each animal from the day before and after treatment. The values are expressed as mean ± SD and ± SEM from eight animals per dose group. Statistical evaluation is performed by Kruskal-Wallis one-way ANOVA using the percent basal values. If statistical significance is reached at the level of $p<0.05$, Mann-Whitney U-test for statistical comparison between control and treatment groups is performed.

The compounds according to the invention show an effect in the range of 5-200 mg/kg.

**Table 7. Biological data**

| **In vitro binding at the human 5-$HT_6$ receptor** | |
|---|---|
| **EXAMPLE** | **$K_i$(nM) human 5-$HT_6$** |
| **1** | 10 |
| **11** | 6.5 |
| **20** | 10.5 |

**In vivo efficacy data**

| **EXAMPLE** | % FI reduction* | Css, u (uM) |
|---|---|---|
| **1** | 12 | 0.44 |

(continued)

| EXAMPLE | % FI reduction* | Css, u (uM) |
|---|---|---|
| **11** | 47.1 | 0.02 |
| *Effect on Food Intake reduction In Ob/ob mice Single administration 50 mg/kg/d measured at steady state | | |

## Claims

**1.** A compound of the formula (I):

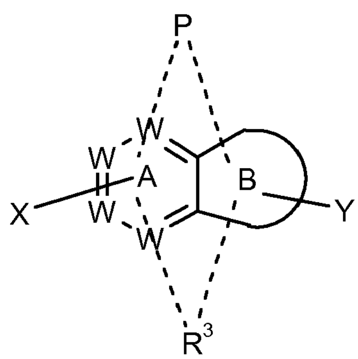

(I)

or a pharmaceutically acceptable salt thereof, wherein:
ring B is

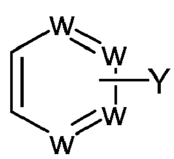

;

each W is independently -N-, -(CH)-, or -C- provided that not more than three groups W are -N- in both rings A and B together, and that rings A and B are not both phenyl;
P is any one of formula (a), (b) or (c)

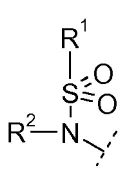

,

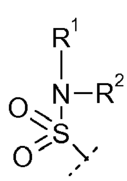

, or

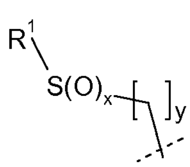

;

(a) (b) (c)

wherein x = 0, 1, or 2 and y = 0, 1, or 2;
and P and $R^3$ can be attached to any carbon atom that allows the substitution in one of either the A- or B-ring, or

when ring A contains at least one nitrogen atom and P is (c), then P can also be attached to any nitrogen in ring B that allows the substitution;
the dashed bonds denote that P and $R^3$, respectively, may be attached to either the A or B ring; but each P or $R^3$ may not be simultaneously bound to both rings A and B;
$R^1$ is

(a) $C_{1-6}$ alkyl,
(b) $C_{1-6}$ alkoxyalkyl,
(c) straight-chained or branched $C_{1-6}$ hydroxyalkyl,
(d) straight-chained or branched $C_{1-6}$ alkylhalides,
(e) aryl carbonylmethyl,
(f) $C_{3-7}$ cycloalkyl, which is optionally partially unsaturated,
(g) $C_{3-7}$ cycloalkyl-$C_{1-6}$ alkyl, wherein the cyclic ring is optionally partially unsaturated, or
(h) a group Ar;

wherein Ar is

(a) phenyl,
(b) 1-naphthyl,
(c) 2-naphthyl,
(d) aryl-$C_{1-6}$ alkyl,
(e) cinnamyl,
(f) a 5 to 7-membered, optionally aromatic, partially saturated or completely saturated, mono- or bi-cyclic heterocyclic ring, each containing 1 to 4 heteroatoms, selected from oxygen, sulfur, and nitrogen,
(g) a bicyclic ring system comprising at least one heterocyclic ring according to (f) and a group Ar,

wherein the group Ar is substituted in one or more positions with

(a) H, X or Y, or
(b) a 5 to 7-membered, optionally aromatic, partially saturated or completely saturated, heterocyclic ring each containing 1 to 4 heteroatoms selected from oxygen, nitrogen or sulfur;

$R^2$ is

(a) H,
(b) $C_{1-6}$ alkyl,
(c) $C_{2-6}$ alkoxyalkyl,
(d) straight or branched $C_{1-6}$ hydroxyalkyl, or
(e) straight or branched $C_{1-6}$ alkylhalides;
(f) a group Ar,

or $R^1$ and $R^2$ are linked to form a group $-CH_2CH_2OCH_2CH_2-$ or

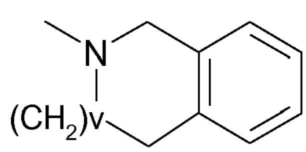

wherein v is 0-2,
X and Y are independently

(a) H,
(b) halogen,
(c) $C_{1-6}$ alkyl,
(d) $CF_3$,
(e) hydroxy,

(f) $C_{1-6}$ alkoxy,
(g) $C_{2-6}$ alkenyl,
(h) phenyl,
(i) phenoxy,
(j) benzyloxy,
(k) benzoyl,
(l) $-OCF_3$,
(m)-CN,
(n) straight or branched $C_{1-6}$ hydroxyalkyl,
(o) straight or branched $C_{1-6}$ alkylhalides,
(p) $-NH_2$,
(q) $-NHR^4$,
(r) $-NR^4R^5$,
(s) $-NO_2$,
(t) $-CONR^4R^5$,
(u) $-NHSO_2R^4$,
(v) $-NR^4COR^5$,
(x) $-SO_2NR^4R^5$,
(z) $-C(=O)R^4$,
(aa) $-CO_2R^4$, or
(ab) $-S(O)_nR^4$, wherein n is 0, 1, 2 or 3,
(ac) -S-($C_{1-6}$) alkyl, or
(ad) $-SCF_3$; and

$R^4$ and $R^5$ are independently

(a) H,
(b) $C_{1-6}$ alkyl,
(c) $C_{3-7}$ cycloalkyl, or
(d) Ar, as defined above for $R^1$;

alternatively, $R^4$ and $R^5$ are linked to form a group $-CH_2OCH_2-$, $-CH_2CH_2OCH_2CH_2-$ or $(CH_2)_{3-5}$;
$R^3$ is a group selected from any one of

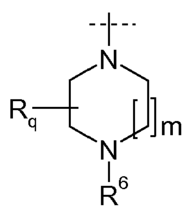
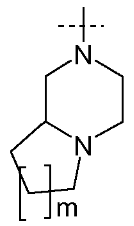
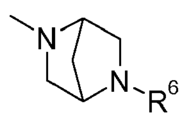
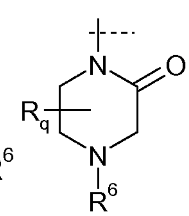
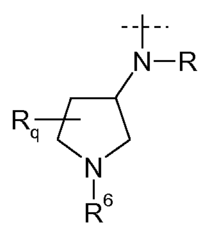

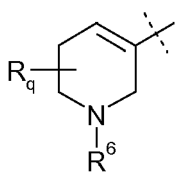
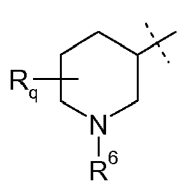
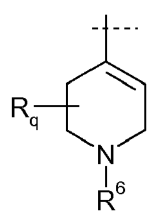
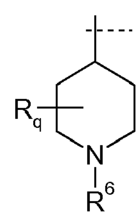
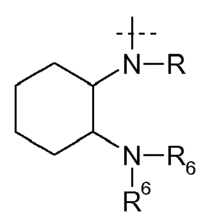

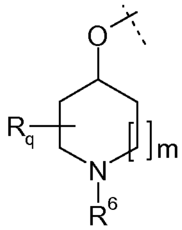
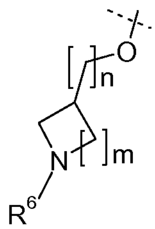
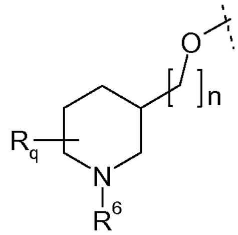
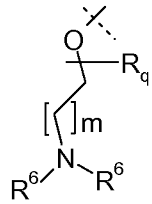

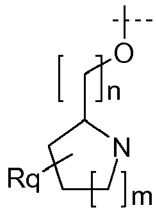
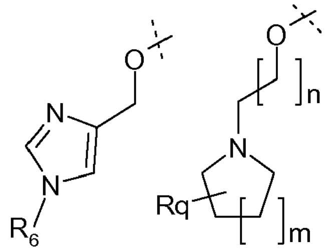
and
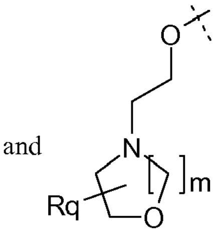

wherein $R^3$ is optionally substituted on each carbon atom that allows the substitution with Rq groups, wherein Rq is independently H, or $(C_{1-6})$ alkyl, and wherein two Rq groups can be present on the same carbon atom simultaneously, wherein
q = 1, 2, 3, 4, 5 or 6,
m = 1 or 2, and
n = 0, 1 or 2;
$R^6$ is independently

(a) H,
(b) linear or branched $C_{1-6}$ alkyl,
(c) benzyl,
(d) $-CH_2-CH_2-OH$, or
(e) $-CH_2-CH_2-O-C_{1-6}$ alkyl;

P and $R^3$ can be attached to the same ring or to different rings of rings A and B;
provided that
when P is

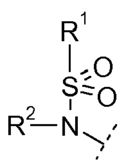 (a) or 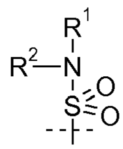 (b),

and P and $R^3$ both are attached to ring A in the meta- or para-position relative to one another then $R^3$ is selected from any one of

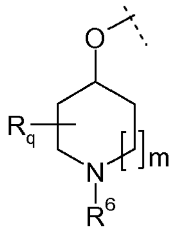
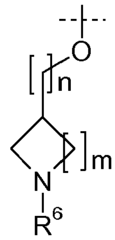
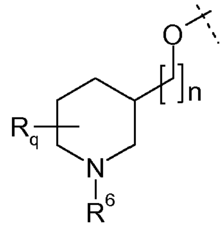
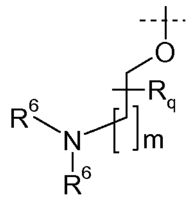

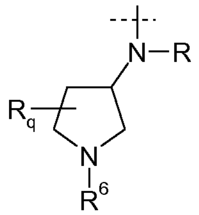
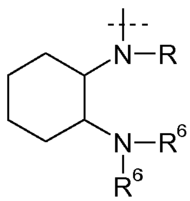
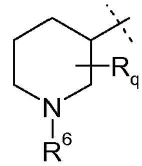
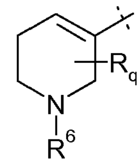

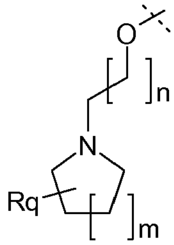
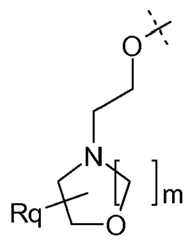
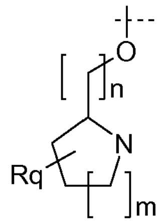
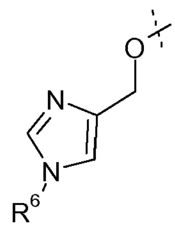
;

when P is

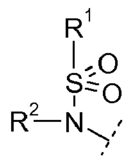
(a),

then P and $R^3$ are simultaneously attached to the same ring A or B;
when P is

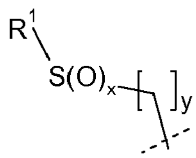
,

wherein y = 0, then P and $R^3$ are attached to the different rings of rings A and B; or
when $R^1$ = Ar is partially saturated bi-cyclic heterocyclic ring containing a N atom, the N atom in Ar cannot be attached to the S atom in P.

**2.** The compound according to claim 1, wherein
$R^1$ is

(a) $C_{1-6}$ alkyl, or
(e) a group Ar;

Ar is

(a) phenyl,
(b) 1-naphthyl,
(c) 2-naphthyl, or
(f) a 5 to 7-membered, optionally aromatic, partially saturated or completely saturated, heterocyclic ring containing 1 to 4 heteroatoms, selected from oxygen, nitrogen and sulfur,

wherein the group Ar is substituted in one or more positions with

(a) H,
(b) halogen,
(c) $C_{1-6}$ alkyl,
(d) $-CF_3$,
(f) $C_{1-6}$ alkoxy,
(g) $C_{2-6}$ alkenyl,
(l) $-OCF_3$,
(m) straight or branched $C_{1-6}$ hydroxyalkyl,
(n) phenyloxy,
(o) benzyloxy,
(v) $-NR^4COR^5$,
(x) $-SO_2NR^4R^5$,
(z) $-C(=O)R^4$,
(ab) $-S(O)_nR^4$, wherein n is 0, 1, 2 or 3;
(ac) $-S-(C_{1-6})$ alkyl, or
(ad) $-SCF_3$;

$R^2$ is

(a) H, or
(b) $C_{1-6}$ alkyl;

or $R^1$ and $R^2$ are linked to form a group $-CH_2CH_2OCH_2CH_2-$;
X and Y are H;
$R^4$ and $R^5$ are each independently H or $C_{1-3}$ alkyl; and
$R^3$ is selected from any one of

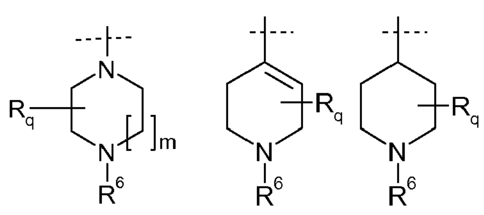

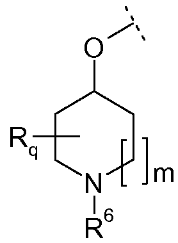 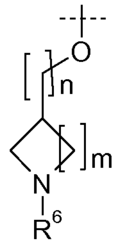 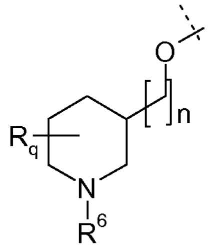 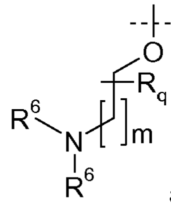 and 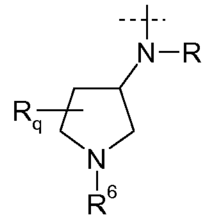

wherein $R^3$ can be substituted on each carbon atom that allows the substitution with Rq groups, wherein Rq is independently H, or $C_{1-6}$ alkyl, and wherein two Rq groups can be present on the same carbon atom simultaneously, wherein

q = 1 or 2,
m = 1 or 2,
n = 0, and
$R^6$ is independently

(a) H,
(b) $C_{1-6}$ alkyl, in particular methyl,
(d) $-CH_2-CH_2-OH$, or
(e) $-CH_2-CH_2-OCH_3$.

**3.** The compound according to claims 1 or 2 wherein $R^3$ is selected from any one of

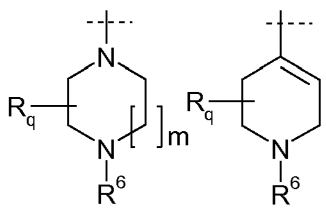 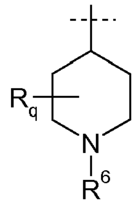 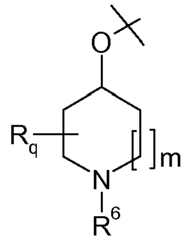 and 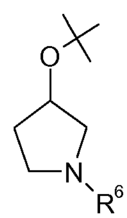

wherein $R^3$ can be substituted on each carbon atom that allows the substitution with Rq groups, wherein Rq is independently H, or $C_{1-2}$ alkyl, and wherein two Rq groups can be present on the same carbon atom simultaneously, wherein

q = 1 or 2,
m = 1 or 2; and
$R^6$ is independently

(a) H,
(b) $C_{1-3}$ alkyl,
(d) $-CH_2-CH_2-OH$, or
(e) $-CH_2-CH_2-OCH_3$.

**4.** The compound according to claim 1 or 2 wherein $R^3$ is selected from any one of

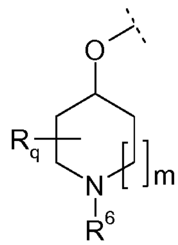 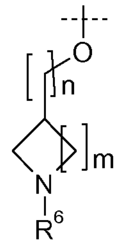 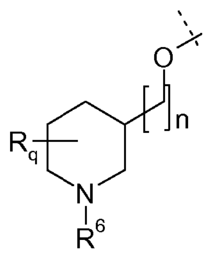 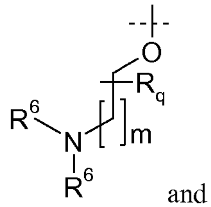 and 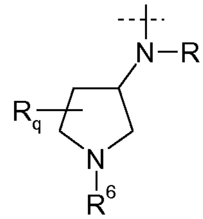

wherein $R^3$ can be substituted on each carbon atom that allows the substitution with Rq groups, wherein Rq is independently H, or $C_{1-6}$ alkyl, and wherein two Rq groups can be present on the same carbon atom simultaneously, wherein
q = 1 or 2,
m = 1 or 2,
n = 0, and
$R^6$ is independently

(a) H,
(b) $C_{1-3}$ alkyl,
(d) $-CH_2-CH_2-OH$, or
(e) $-CH_2-CH_2-OCH_3$.

**5.** The compound according to claim 1 or 2 wherein $R^3$ is selected from any one of

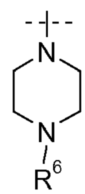 ; 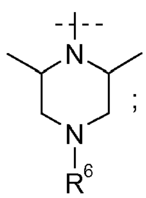 ; 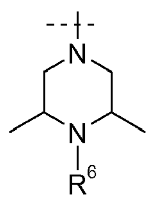 ; 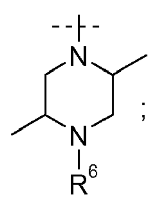 ; 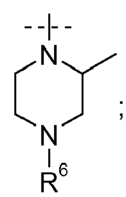 ;

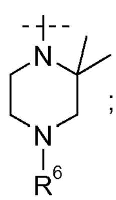 ; 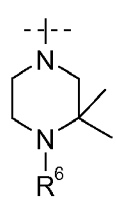 ; 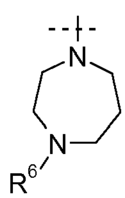 ; 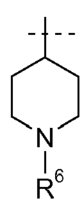 ; 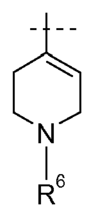 ;

$R^6$ is independently

(a) H,
(b) $C_{1-3}$ alkyl,
(d) $-CH_2-CH_2-OH$, or
(e) $-CH_2-CH_2-OCH_3$.

**6.** A compound according to any one of claims 1 to 5 wherein $R^6$ is H or methyl.

**7.** A compound according to claim 1 or 2 wherein $R^3$ is piperazine; homopiperazine; 2,6-dimethylpiperazine; 3,5-dimethylpiperazine; 2,5-dimethylpiperazine; 2-methylpiperazine; 3-methylpiperazine; 2,2-dimethylpiperazine; 3,3-dimethylpiperazine; piperidine; 1,2,3,6-tetrahydro-pyrazine; or 4-pyrrolidin-3-yloxy.

**8.** The compound according to any one of claims 1 to 7 wherein the groups Y and X are attached to any unsubstituted carbon atom.

**9.** The compound according to any one of claims 1 to 8, wherein P is

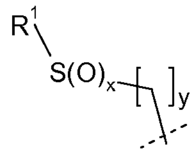

(c)

wherein $R^1$, x, and y are as defined in claim 1.

**10.** The compound according to claim 9 of the general formula (II)

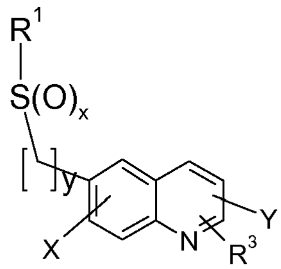

(II)

wherein $R^1$, x, y, X, and Y are as defined in claim 1, and $R^3$ is as defined in claim 2.

**11.** The compound according to claim 10 wherein y = 0 and x = 2

**12.** The compound according to claim 9 of the general formula (III)

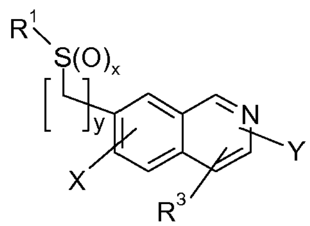

(III)

wherein $R^1$, x, y, X, and Y are as defined in claim 1, and $R^3$ is as defined in claim 2.

**13.** The compound according to claim 12 wherein y = 0 and x = 2

**14.** The compound according to claim 10, which is the compound 6-Benzenesulfonyl-4-piperazin-1-yl-quinoline hydrochloride; 6-[(2-Fluorophenyl)sulfonyl]-4-piperazin-1-ylquinoline hydrochloride; 6-(1-Naphthylsulfonyl)-4-piperazin-1-ylquinoline hydrochloride; 6-[(3,4-Dichlorophenyl)sulfonyl]-4-piperazin-1-ylquinoline hydrochloride; 6-[(3,5-Dimethylphenyl)sulfonyl]-4-piperazin-1-ylquinoline hydrochloride; 6-[(2-Chloro-6-methylphenyl)sulfonyl]-4-piperazin-1-ylquinoline hydrochloride; 6-[(4-Chlorophenyl)sulfonyl]-4-piperazin-1-ylquinoline hydrochloride; 6-[(2-Methyl, 4-tert-butyl-phenyl)sulfonyl]-4-piperazin-1-ylquinoline hydrochloride; 6-[(3,4-Dimethylphenyl)sulfonyl]-4-piperazin-1-ylquinoline hydrochloride; 6-[(2,3-Dichlorophenyl)sulfonyl]-4-piperazin-1-ylquinoline hydrochloride; 6-[(4-tert-Butylphenyl)sulfonyl]-4-piperazin-1-ylquinoline hydrochloride; 6-[(4-Isopropylphenyl)sulfonyl]-4-piperazin-1-ylquinoline hydrochloride; (4-Piperazin-1-yl-6-{[4-(trifluoromethyl)phenyl]sulfonyl}quinoline hydrochloride; 6-[(4-tert-Butylphenyl)sulfonyl]-4-(1,4-diazepan-1-yl)quinoline hydrochloride; or 4-(1,4-Diazepan-1-yl)-6-[(4-isopropylphenyl)sulfonyl]quinoline hydrochloride.

**15.** The compound according to claim 13, which is the compound 7-(2-Chloro-6-methyl-benzenesulfonyl)-1-piperazin-1-yl-isoquinoline hydrochloride; 7-(2-*t*-Butyl-benzenesulfonyl)-1-piperazin-1-yl-isoquinoline hydrochloride; 7-(3,4-Dichloro-benzenesulfonyl)-1-piperazin-1-yl-isoquinoline hydrochloride; 7-(2,4-Dimethyl-benzenesulfonyl)-1-piperazin-1-yl-isoquinoline hydrochloride; 7-(2,5-Dimethyl-benzenesulfonyl)-1-piperazin-1-yl-isoquinoline hydrochloride; 7-(*p*-Chloro-benzenesulfbnyl)-1piperazin-1-yl-isoquinoline hydrochloride; 7-Benzenesulfonyl-1-[1,4]diazepan-1-yl-isoquinoline,hydrochloride; 7-(4-tert-Butyl-benzenesulfonyl)-1-[1,4]diazepan-1-yl]-isoquinoline, hydrochloride; 7-(2-Chloro-6-methyl-benzenesulfonyl)-1-[1,4]diazepan-1-yl]-isoquinoline hydrochloride; 7-(3,5-Dimethyl-benzenesulfonyl)-1-[1,4]diazepan-1-yl]-isoquinoline hydrochloride; 7-(3,4-Dichloro-benzenesulfonyl)-1-[1,4]diazepan-1-yl]-isoquinoline hydrochloride; 7-(4-Chloro-benzenesulfonyl)-1- [1,4]diazepan-1-yl]-isoquinoline hydrochloride; 7-(3,4-Dimethyl-benzenesulfonyl)-1-[1,4]diazepan-1-yl]-isoquinoline hydrochloride; 7-(2-tert-Butyl-benzenesulfonyl)-1-[1,4]diazepan-1-yl]-isoquinoline hydrochloride; 7-Benzenesulfonyl-1-piperazin-yl-isoquinoline hydrochloride; or 7-(4-tert-Butyl-benzenesulfonyl-1-piperazin-yl-isoquinoline hydrochloride

**16.** A compound according to any one of claims 1 to 15 for use in therapy.

**17.** A pharmaceutical formulation comprising a compound according to any one of claims 1 to 15 as an active ingredient, in combination with a pharmaceutically acceptable diluent or carrier.

**18.** Use of a compound according to any one of claims 1 to 15 for the manufacture of a medicament for use in the treatment or prophylaxis of a 5-$HT_6$ receptor related disorder, such as obesity, type II diabetes, and/or disorders of the central nervous system, to achieve reduction of body weight and of body weight gain.

**19.** Use of a compound according to any one of claims 1 to 15 for the manufacture of a medicament for use in the treatment or prophylaxis of disorders of the central nervous system.

**20.** Use of a compound according to any one of claims 1 to 15 for the manufacture of a medicament for use in the treatment or prophylaxis of type II diabetes.

**21.** Use of a compound according to any one of claims 1 to 15 for the manufacture of a medicament for use in the treatment or prophylaxis of obesity, to achieve reduction of body weight and of body weight gain.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0034242 A
- WO 9942465 A
- WO 0132646 A1
- WO 9937623 A2
- WO 9942465 A3
- EP 0815861 A1
- WO 9902502 A2
- WO 9827081 A1
- EP 0701819 A
- US 6191141 B
- WO 0112629 A

### Non-patent literature cited in the description


- **RUAT, M. et al.** *Biochem. Biophys. Res. Commun.,* 1993, vol. 193, 268-276
- **SEBBEN, M. et al.** *NeuroReport,* 1994, vol. 5, 2553-2557
- **BENTLEY, J.C. et al.** *Br J Pharmac. Suppl.,* 1999, vol. 126, 66
- **BENTLEY, J.C. et al.** *J. Psychopharmacol. Suppl.,* 1997, vol. A64, 255
- **WOOLLEY M.L. et al.** *Neuropharmacology,* 2001
- **ISAAC, M. et al.** 6-Bicyclopiperazinyl-1-arylsulfonylindoles and 6-Bicyclopiperidinyl-1-arylsulfonylindoles derivatives as novel, potent and selective 5-HT6 receptor antagonists. *Bioorganic & Medicinal Chemistry Letters,* 2000, vol. 10, 1719-1721
- *J. Med. Chem.,* 1970, vol. 13 (4), 592-598
- **R. LAROCK.** Comprehensive Organic Transformations. VCH Publishers, 1989
- **T.W. GREENE ; P.G.M. WUTS.** Protective Groups in Organic Synthesis. John Wiley and Sons, 1991
- **L. FIESER ; M. FIESER.** Fieser and Fieser's Reagents for Organic Synthesis. John Wiley and Sons, 1994
- Encyclopedia of Reagents for Organic Synthesis. John Wiley and Sons, 1995
- **BOESS F.G et al.** *Neuropharmacology,* 1997, vol. 36 (4/5), 713-720
- **BRADFORD MM.** A rapid and sensitive method for the quantitation of microgram quantities of protein utilizing the principle of protein-dye binding. *Anal Biochem,* 1976, vol. 72, 248-54
- **CHENG Y.C.** *Biochem. Pharmacol.,* 1973, vol. 22, 3099-3108
- **BOESS et al.** *Neuropharmacology,* 1997, vol. 36, 713-720
- **BLUNDELL, J.E. ; HALFORD, J.C.G.** Serotonin and Appetite Regulation. Implications for the Pharmacological Treatment of Obesity. *CNS Drugs,* 1998, vol. 9, 473-495
- **THEEUWES, F. ; YAM, S.I.** *Ann. Biomed. Eng.,* 1976, vol. 4 (4), 343-353